# FASCICULE DE BREVET EUROPEEN

(11) **EP 0 864 561 B1**
(45) Date de publication et mention de la délivrance du brevet: **05.09.2001**
(21) Numéro de dépôt: 98400468.9
(22) Date de dépôt: 27.02.1998
(51) Int. Cl.: C07C 311/20, C07C 381/00, C07C 311/19, A61K 31/18

(54) **Nouveaux dérivés de benzènesulfonylamine, leur procédé de préparation et les compositions pharmaceutiques qui les contiennent**
Benzolsulfonylamin-Derivate, Verfahren zu ihrer Herstellung und diese enthaltende pharmazeutische Zusammensetzungen
Benzenesulfonylamine derivatives,process for their preparation and pharmaceutical compositions containing them

(30) Priorité: 28.02.1997 FR 9702445
(43) Date de publication de la demande: 16.09.1998
(73) Titulaire: ADIR ET COMPAGNIE, 92415 Courbevoie Cédex (FR)
(72) Inventeur: Lavielle, Gilbert, 78170 La Celle Saint Cloud (FR); Cimetiere, Bernard, 75020 Paris (FR); Verbeuren, Tony, 78540 Vernouillet (FR); Simonet, Serge, 78700 Conflans Saint Honorine (FR); Descombes, Jean-Jacques, 92500 Rueil-Malmaison (FR)

(56) Documents cités:
- EP-A- 0 031 954
- EP-A- 0 589 037
- EP-A- 0 648 741
- WO-A-94/12488

## Description

La présente invention concerne de nouveaux dérivés de benzènesulfonylamine, leur procédé de préparation et les compositions pharmaceutiques qui les contiennent.

Ces composés, outre le fait qu'ils soient nouveaux, possèdent des propriétés anti-thromboxane A₂ en tant qu'antagoniste des récepteurs au thromboxane A₂ (TXA₂) et en même temps des propriétés donneur de NO.

Le TXA₂ est un métabolite de l'acide arachidonique produit par les plaquettes sanguines et par certaines autres cellules de l'organisme, qui provoque une constriction importante des vaisseaux sanguins, induit l'agrégation plaquettaire et possède des propriétés prolifératives. La production du TXA₂ est entre autre augmentée dans des maladies coronariennes (e.g. angine de poitrine, infarctus du myocarde) et dans les accidents vasculaires cérébraux et le TXA₂ joue un rôle capital dans tous les processus conduisant aux maladies thrombotiques. En plus, ce médiateur est impliqué dans de nombreuses autres pathologies humaines telles que le diabète, l'hypertension et l'athérosclérose (J.C. Ansquer ; STV ; 4 ; 161-167, 1992).

Il est donc intéressant de synthétiser des substances susceptibles d'éliminer les activités proagrégantes, vasoconstrictrices et prolifératives du TXA₂ ; vu le fait que ces actions se produisent par l'intermédiaire de récepteurs membranaires spécifiques (des TP-récepteurs), des molécules antagonistes des TP-récepteurs ont été synthétisées.

Le monoxyde d'azote (NO) a été découvert récemment comme une molécule impliquée dans des réactions physiologiques très diverses ; le NO est un vasodilatateur produit par les cellules endothéliales, un anti-agrégant plaquettaire produit par les plaquettes, un neurotransmetteur présent dans des neurones nitridergiques et une substance dont la production (par des NO-synthases) est induite dans l'organisme suite à des stimuli inflammatoires. En plus, le NO possède également des propriétés antiprolifératives (Moncada and Higgs, N. Engl. J. Med., 329, 2002-2012, 1993).

Des dérivés nitrés, tels que la nitroglycérine, ont été utilisés dans la thérapie des maladies coronariennes depuis plusieurs décennies. L'action de ses dérivés repose sur le fait que ces produits sont des donneurs de NO dont la production et/ou la libération est compromise dans les maladies. Les substances donneurs de NO sont des vasodilatateurs et également des antiagrégants plaquettaires (J.S. Stamler, JACC, 1529-1536, 1991; I. Salvemini et al., Biochem. Pharmacol., 44, 17-24, 1992). Des substances donneurs de NO sont donc utiles pour la pathologie cardiovasculaire impliquant des vasospasmes et/ou de l'agrégation plaquettaire et/ou de la prolifération.

Il était donc particulièrement intéressant de rechercher des substances ayant les deux propriétés. En effet, inhiber les effets d'une substance vasoconstrictrice, proagrégante. prolifératrice (le TXA₂) libérée en excès dans les maladies décrites et remplacer une substance vasodilatatrice, anti-agrégante, anti-proliférative (NO) dont la production et/ou la libération est compromise est une stratégie particulièrement intéressante conduisant à des produits qui seront capables de restaurer un malfonctionnement au maximum. Cette stratégie conduit en effet à la découverte de substances ayant deux activités indépendantes mais synergistiques au niveau des plaquettes et des vaisseaux sanguins. Un avantage considérable de cette stratégie de synergie est que les doses du donneur de NO peuvent être relativement faible afin d'éviter les conséquences hémodynamiques trop importantes normalement liées à un traitement aux dérivés nitrés.
Les produits sont donc utiles en tant qu'antagonistes des TP-récepteurs et donneurs de NO dans le traitement et/ou la prévention des maladies dans lesquelles le TXA₂ est impliqué et les taux et/ou l'activité du NO sont diminués comme p.e. les maladies cardio et cérébrovasculaires, les maladies thrombotiques ainsi que les complications vasculaires qui accompagnent les conditions pathologiques liés à une production ou une activité exagérée de TXA₂ et/ou une production ou activité diminuée du NO (e.g. la resténose, les complications vasculaires dans le diabète, l'hypertension, l'athérosclérose, la maladie de Raynaud, la maladie rénale, la maladie pulmonaire). En tant qu'antagoniste des TP-récepteurs et donneurs de NO, ces produits possèdent également des propriétés protectrices de la paroi gastrique (M.L. Ogletree et coll., J. Pharm. Exp. Ther. : 263 : 374-380, 1992 ; S.J. Konturek et al.. Scand. J. Gastroenterol. : 30 : suppl. 220 : 22-27, 1995) et peuvent être bénéfiques dans l'impuissance (Moncada and Higgs : N. Engl. J. Med : 329 : 2002-2012, 1993).
Les antagonistes des TP-récepteurs peuvent également être utilisés dans l'asthme (E. Samara, Cardiovasc. Drug. Rev. 14 : 272-282, 1996). Grâce aux propriétés inhibitrices de l'agrégation plaquettaire, les composés de l'invention peuvent également être proposés dans le traitement de la migraine (P. Puig-Parellada et al., Prostagland. Leukotr. Essentail Fatty acids : 49 : 537-547, 1993).

Plus particulièrement, la présente invention concerne les composés de formule (I) : 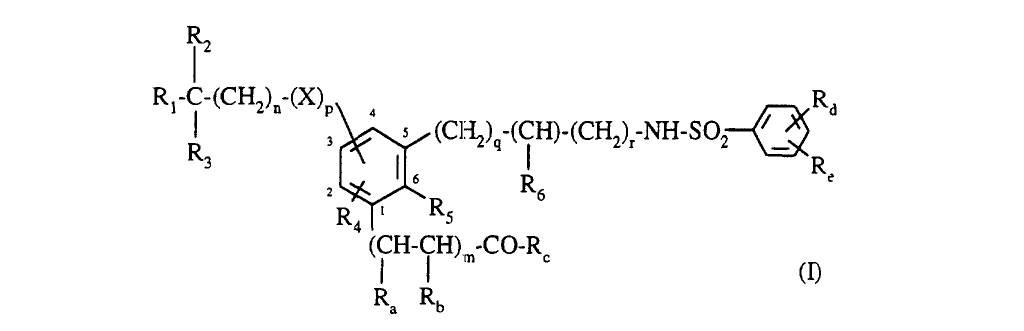 dans laquelle :
- Rₐ, R_{b},: identiques ou différents, représentent un atome d'hydrogène ou un groupement alkyle (C₁-C₆) linéaire ou ramifié,
ou bien Rₐ, R_{b} forment ensemble une liaison,
- R_{c}: représente un groupement hydroxy, alkoxy (C₁-C₆) linéaire ou ramifié (substitué éventuellement par un groupement -O-NO₂, -O-NO ou -S-NO), un groupement amino (substitué éventuellement par un ou deux groupements, identiques ou différents, alkyle (C₁-C₆) linéaire ou ramifié, phényle éventuellement substitué),
- R₁: représente un atome d'hydrogène ou un groupement -O-NO₂, -O-NO ou -S-NO,
- R₂, R₃,: identiques ou différents, représentent un atome d'hydrogène ou un groupement alkyle (C₁-C₆) linéaire ou ramifié, ou phényle éventuellement substitué.
- X: représente un atome d'oxygène ou un groupement -NH-CO-,
- m: représente 0 ou 1,
- n: représente un nombre entier tel que 0 ≤ n ≤ 6,
- p: représente 0 ou 1,
- R₄: représente un atome d'hydrogène, un groupement alkyle (C₁-C₆) linéaire ou ramifié, (éventuellement substitué par un groupement hydroxy), phényle éventuellement substitué ou un groupement : 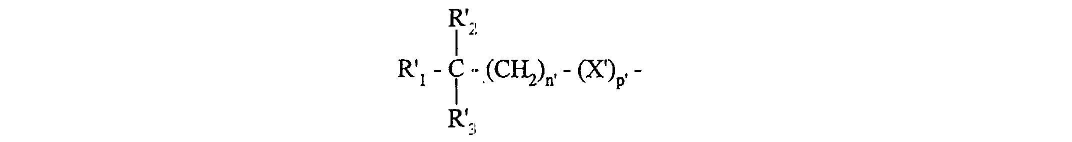 dans lequel :
R'₁ représente un atome d'hydrogène ou un groupement : -O-NO₂, -O-NO ou -S-NO,
R'₂, R'₃, identiques ou différents, représentent un atome d'hydrogène ou un groupement alkyle (C₁-C₆) linéaire ou ramifié, ou phényle éventuellement substitué,
X' représente un atome d'oxygène ou un groupement -NH-CO-,
n' représente un nombre entier tel que 0 ≤ n ≤ 6,
p' représente 0 ou 1,
- R₅: représente un atome d'hydrogène ou un groupement alkyle (C₁-C₆), linéaire ou ramifié,
- R₆: représente un atome d'hydrogène ou un groupement alkyle (C₁-C₆) linéaire ou ramifié, ou bien R₅ et R₆ forment ensemble une chaîne -(CH₂)ₜ- dans laquelle t représente 1 ou 2,
- q: représente 0, 1 ou 2,
- r: représente un nombre entier tel que 0 ≤ r ≤ 6,
- R_{d}, Rₑ,: identiques ou différents, représentent un atome d'hydrogène, un atome d'halogène, un groupement alkyle (C₁-C₆) linéaire ou ramifié, un groupement alkoxy (C₁-C₆) linéaire ou ramifié, un groupement hydroxy ou un groupement trihalogénoalkyle (C₁-C₆) linéaire ou ramifié,
étant entendu qu'au moins un groupement -O-NO₂, -O-NO ou -S-NO soit présent en R₁, R₄ ou R_{c},
et leurs sels d'addition à un acide ou à une base pharmaceutiquement acceptable.

Les composés préférés de l'invention sont ceux pour lesquels, pris ensemble ou séparément :
- Rₐ et R_{b} représentent chacun un atome d'hydrogène et m est égal à 1,
- R₁ représente un groupement -O-NO₂, -O-NO, ou -S-NO,
- R₄ représente un atome d'hydrogène,
- R₅ et R₆ forment ensemble une chaîne -(CH₂)ₜ,
- R_{c} représente un groupement hydroxy ou un groupement alkoxy (C₁-C₆) linéaire ou ramifié,
- R_{d} représente un atome d'halogène,
- p est égal à 0,
- r est égal à 0,

Plus préférentiellement l'invention concerne les composés de formule (I) pour lesquels R₁ représente un groupement -O-NO₂ tandis que p est égal à 0, Rₐ et R_{b} représentent chacun un atome d'hydrogène tandis que m est égal à 1, R₄ représente un atome d'hydrogène, R₅ et R₆ forment ensemble une chaîne -(CH₂)ₜ avec t égal à 2 tandis que q est égal à 1, et r est égal à 0, Rd représente un atome d'halogène tandis que Re représente un hydrogène, et Rc représente un groupement hydroxy ou un groupement alkoxy (C₁-C₆) linéaire ou ramifié.

Par phényle éventuellement substitué, on entend éventuellement substitué par un ou plusieurs, identiques ou différents, atomes d'halogène ou groupements alkyle (C₁-C₆) linéaire ou ramifié, alkoxy (C₁-C₆) linéaire ou ramifié, hydroxy ou trihalogénoalkyle (C₁-C₆) linéaire ou ramifié.

L'invention s'étend également au procédé de préparation des composés de formule (I).

Le procédé de préparation des dérivés de formule (I) tels que p=p'=0 est caractérisé en ce que l'on utilise comme produit de départ un composé de formule (II) : 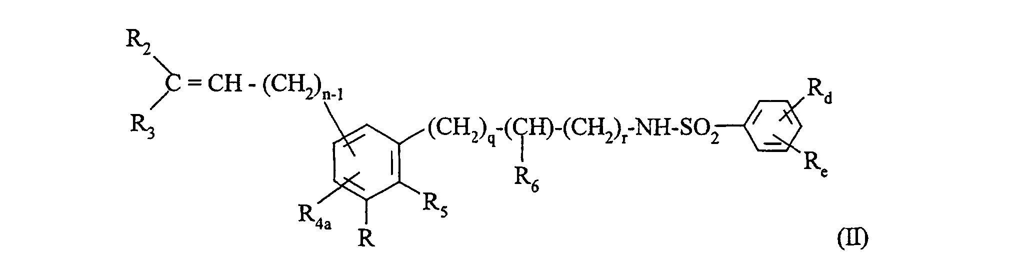 dans laquelle :
R_{d}, Rₑ, R₂, R₃, R₅, R₆, n, q, r ont la même signification que dans la formule (I),
R₄ₐ représente un atome d'hydrogène, un groupement alkyle (C₁-C₆) linéaire ou ramifié (éventuellement substitué par un groupement hydroxy), phényle éventuellement substitué ou un groupeme 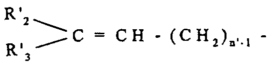 (dans lequel R'₂, R'₃ et n' ont la même signification que dans la formule (I)),
et R représente -CH₂-CH₂-CO₂R'_{c} ou -CO₂R'_{c} (dans lesquels R'_{c} représente un groupement alkyle (C₁-C₆) linéaire ou ramifié éventuellement substitué par un groupement hydroxy),
que l'on soumet à un traitement oxydant approprié de la double liaison en alcool correspondant,
pour conduire au composé de formule (III): 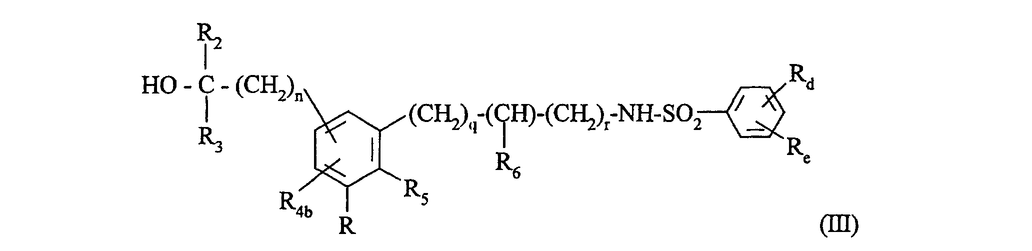 dans laquelle :
R, R_{d}, Rₑ, R₂, R₃, R₅, R₆, n, q et r ont la même définition que précédemment,
R_{4b} représente un atome d'hydrogène, un groupement alkyle (C₁-C₆) linéaire ou ramifié (éventuellement substitué par un groupement hydroxy), phényle éventuellement substitué ou un groupement 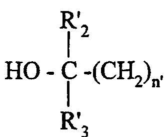 (dans lequel R'₂, R'₃ et n' ont la même signification que précédemment),
composé de formule (III) qui est éventuellement transformé lorsque R représente un groupement -CO₂Alk (Alk étant un groupement alkyle (C₁-C₆) linéaire ou ramifié) en aldéhyde correspondant puis qui subit une réaction de Wittig, pour conduire à un composé de formule (III) dans laquelle le groupement R est remplacé par le groupement -CH=CH-CO₂R'_{c}, R'_{c} étant tel que défini précédemment,
composé de formule (III) :
**soit** dont on transforme le ou les groupements hydroxy en un groupement OTs (dans lequel Ts représente le groupement Tosyle) ou en un atome de brome et qui subit l'action du nitrate de tétrabutylammonium ou du nitrate d'argent,
   pour conduire au composé de formule (I/a), cas particulier des composés de formule (I) : 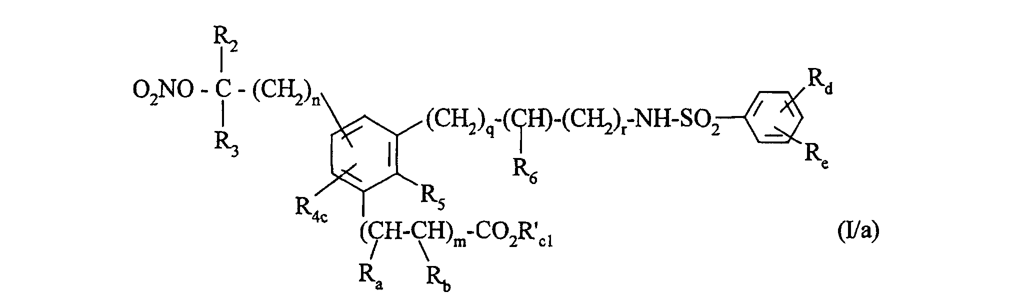 dans laquelle :
   R₂, R₃, R₅, R₆, Rₐ, R_{b}, R_{d}, Rₑ, m, n, q et r ont la même signification que dans la formule (I), et R_{4c} représente un atome d'hydrogène, un groupement alkyle (C₁-C₆) linéaire ou ramifié, phényle éventuellement substitué ou un groupement 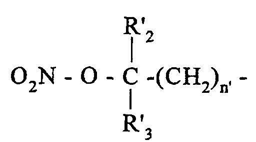 (dans lequel
   R'₂, R'₃ et n' ont la même signification que précédemment et,
      R'c₁ représente un groupement alkyle (C₁-C₆) linéaire ou ramifié (éventuellement substitué par un groupement -O-NO₂)),
**soit** que l'on fait réagir avec du nitrite de terbutyle, pour conduire au composé de formule (I/b), cas particulier des composés de formule (I) : 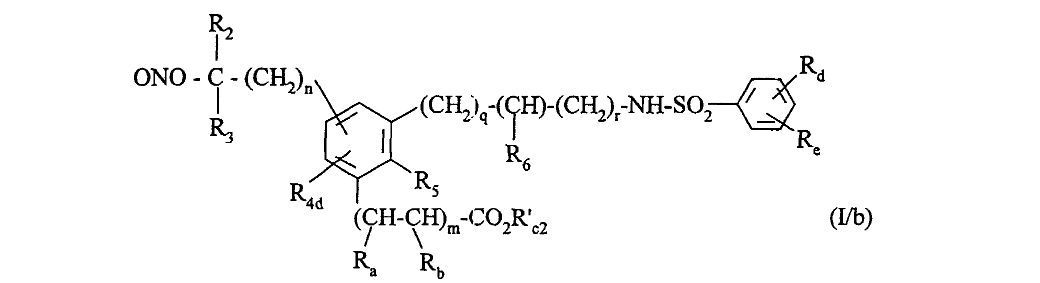 dans laquelle :
   R₂, R₃, R₅, R₆, Rₐ, R_{b}, R_{d}, m, n, q et r ont la même signification que précédemment, et
   R_{4d} représente un atome d'hydrogène, un groupement alkyle (C₁-C₆) linéaire ou ramifié, phényle éventuellement substitué ou un groupement 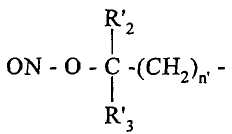 (dans lequel R'₂, R'₃ et n' ont la même signification que précédemment et,
   R'c₂ représente un groupement alkyle (C₁-C₆) linéaire ou ramifié (éventuellement substitué par un groupement -O-NO)),
composé de formule (I/a) ou (I/b), dont on transforme, si on le désire, la fonction ester en fonction acide ou amide correspondante,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou une base pharmaceutiquement acceptable.

Le procédé de préparation des composés de formule (I) tels que p ou p' est égal à 1 et X et X' représentent -NH-CO- est caractérisé en ce que l'on utilise comme produit de départ un composé de formule (IV) : 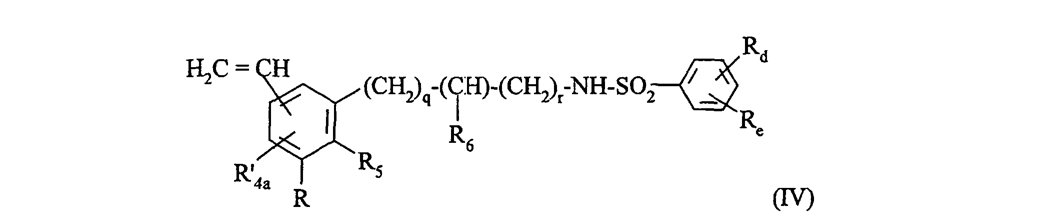 dans laquelle :
R, R_{d}, Rₑ, R₅, R₆, q, r ont la même signification que précédemment et
R'₄ₐ représente un atome d'hydrogène ou un groupement alkyle (C₁-C₆) linéaire ou ramifié (éventuellement substitué par un groupement hydroxy), phényle éventuellement substitué ou un groupement vinyle,
dont on transforme le ou les groupements vinyliques en groupements formyles par coupure oxydative puis en groupements carboxy correspondants,
que l'on fait réagir avec une amine de formule (V): 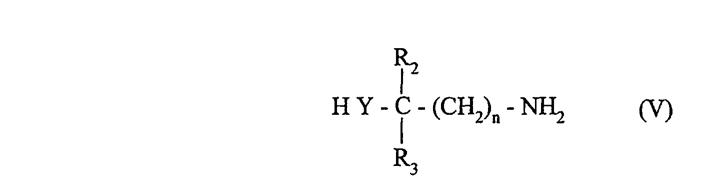 dans laquelle :
R₂, R₃ et n ont la même signification que dans la formule (I)
   et Y représente un atome de soufre ou d'oxygène,
   pour conduire au composé de formule (VI) : 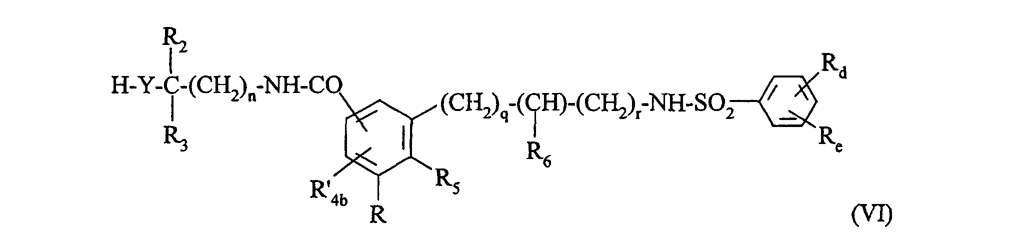 dans laquelle :
R, R_{d}, Rₑ, R₅, R₂, R₃, R₆, Y, n, q et r ont la même signification que précédemment,
R'_{4b} représente un atome d'hydrogène ou un groupement alkyle (C₁-C₆) linéaire ou ramifié (éventuellement substitué par un groupement hydroxy), phényle éventuellement substitué ou un groupement 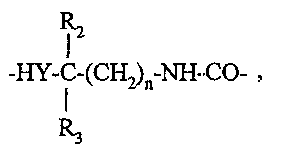
composé de formule (VI) qui est éventuellement transformé lorsque R représente un groupement -CO₂Alk (Alk étant un groupement alkyle (C₁-C₆) linéaire ou ramifié), en aldéhyde correspondant puis qui subit une réaction de Wittig, pour conduire à un composé de formule (VI) dans laquelle le groupement R est remplacé par le groupement -CH=CH-CO₂R'_{c}, R'_{c} étant tel que défini précédemment,
composé de formule (VI) :
**soit** dont on transforme le ou les groupements hydroxy en un groupement OTs (dans lequel Ts représente le groupement Tosyle) ou en atome de brome et qui subit l'action du nitrate de tétrabutylammonium ou du nitrate d'argent,
   pour conduire au composé de formule (I/c), cas particulier des composés de formule (I) : 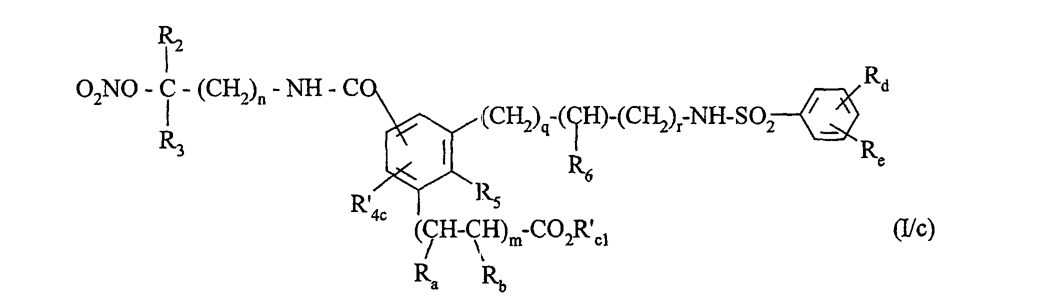 dans laquelle :
   R₂, R₃, R₅, R₆, Rₐ, R_{b}, R_{d}, Rₑ, m, n, q et r ont la même signification que dans la formule (I), et
   R'_{4c} représente un atome d'hydrogène, un groupement alkyle (C₁-C₆) linéaire ou ramifié (éventuellement substitué par un groupement -O-NO₂), phényle éventuellement substitué ou un groupement 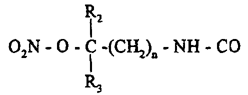 (dans lequel R₂, R₃ et n ont la même signification que précédemment et
   R'c₁ représente un groupement alkyle (C₁-C₆) linéaire ou ramifié (éventuellement substitué par un groupement -O-NO₂)),
**soit** que l'on fait réagir avec du nitrite de terbutyle, pour conduire au composé de formule (I/d), cas particulier des composés de formule (I) : 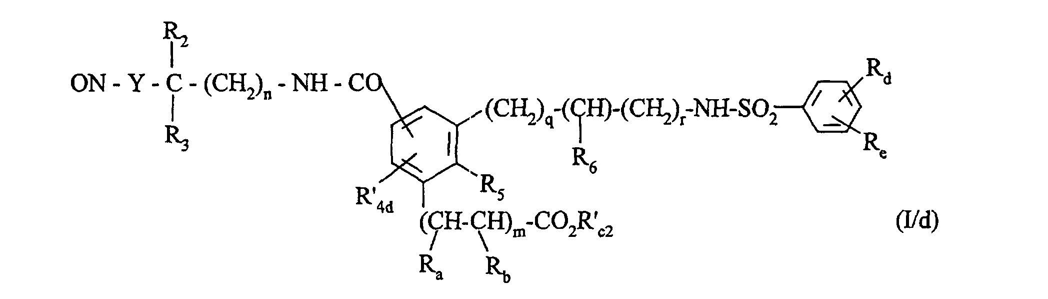 dans laquelle :
   Y, R₂, R₃, R₅, R₆, Rₐ, R_{b}, R_{d}, Rₑ, m, n, q et r ont la même signification que précédemment, et
   R'_{4d} représente un atome d'hydrogène, un groupement alkyle (C₁-C₆) linéaire ou ramifié (éventuellement substitué par un groupement -O-NO), phényle éventuellement substitué ou un groupement 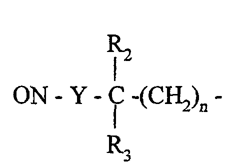 (dans lequel Y, R₂, R₃ et n ont la même signification que précédemment et R'_{c2} représente un groupement alkyle (C₁-C₆) linéaire ou ramifié (éventuellement substitué par un groupement -O-NO)),
composé de formule (I/c) ou (I/d), dont on transforme, si on le désire, la fonction ester en fonction acide ou amide correspondante,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou une base pharmaceutiquement acceptable.

Le procédé de préparation des composés de formule (I) tels que X représente un atome d'oxygène est caractérisé en ce que l'on utilise comme produit de départ un composé de formule (VII) : 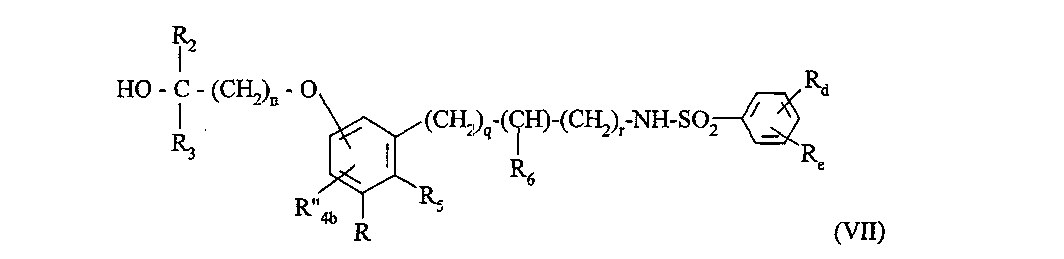 dans laquelle :
R₂, R₃, R₅, R₆, R_{d}, Rₑ, r, q et n sont tels définis dans la formule (I), R représente un groupement 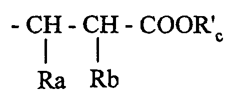 ou COOR'_{c} (dans lesquels R'_{c} représente un groupement alkyle (C₁-C₆) linéaire ou ramifié éventuellement substitué par un groupement hydroxy et Rₐ et R_{b} sont tels que définis dans la formule (I)),
et R"_{4b} représente un atome d'hydrogène, un groupement alkyle (C₁-C₆) linéaire ou ramifié, phényle éventuellement substitué ou un groupement 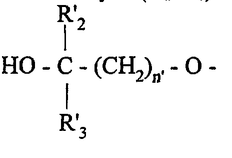 dans lequel n', R'₂ et R'₃ sont tels que définis dans la formule (I)),
composé de formule (VII) :
**soit** dont on transforme le ou les groupements hydroxy en un groupement OTs (dans lequel Ts représente un groupement Tosyle) ou en un atome de brome, et qui subit l'action du nitrate de tétrabutylammonium ou du nitrate d'argent,
   pour conduire à un composé de formule (I/e), cas particulier des composés de formule (I) 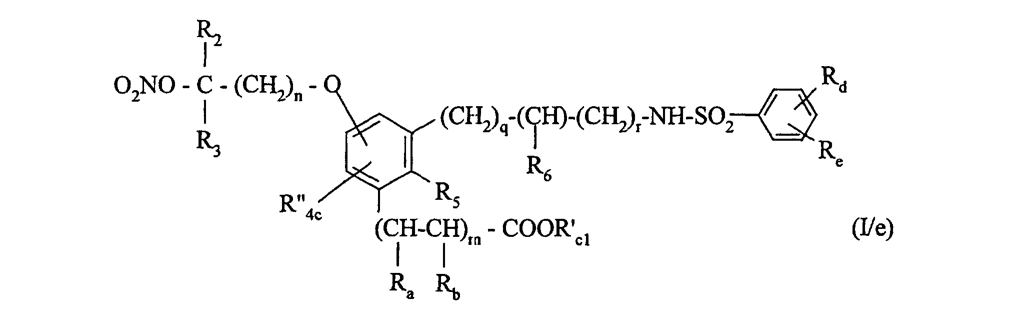 dans laquelle:
   R₂, R₃, R₅, R₆, Rₐ, R_{b}, R_{d}, Rₑ, m, n, q et r ont la même signification que dans la formule (I), et
   R"_{4c} représente un atome d'hydrogène, un groupement alkyle (C₁-C₆) linéaire ou ramifié, phényle éventuellement substitué ou un groupement 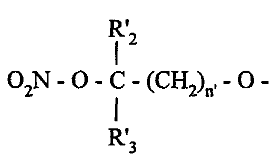 dans lequel R'₂, R'₃, et
   et n' ont la même que précédemment) et R'_{C1} représente un groupement alkyle (C₁-C₆) linéaire ou ramifié éventuellement substitué par un groupement -ONO₂,
**soit** que l'on fait réagir avec du nitrite de tertbutyle pour conduire au composé de formule (I/f), cas particulier des composés de formule (I) : 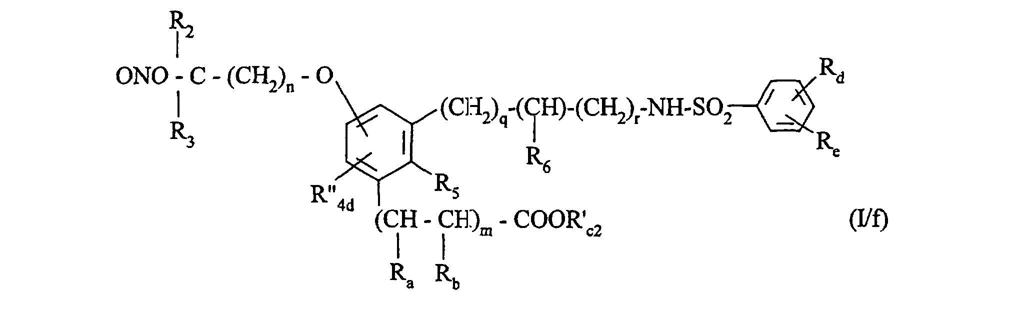 dans laquelle :
   R₂, R₃, R₅, R₆, Rₐ, R_{b}, R_{d}, Rₑ, n, m, q et r ont la même signification que dans la formule (I),
   R"_{4d} représente un atome d'hydrogène, un groupement alkyle (C₁-C₆) linéaire ou ramifié, phényle éventuellement substitué ou un groupement 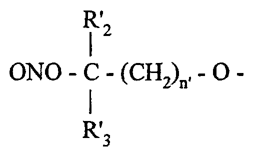 dans lequel R'₂, R'₃ et n' ont la même signification que précédemment,
   et R'_{C2} représente un groupement alkyle (C₁-C₆) linéaire ou ramifié éventuellement substitué par un groupement -O-NO,
   composé de formules (I/e) ou (I/f), dont on transforme, si on le désire, la fonction ester en fonction acide ou amide correspondante,
   - qui peut être, le cas échéant, purifié selon une technique classique de purification,
   - dont on sépare, le cas échéant, les isomères selon une technique classique de séparation,
   - que l'on transforme, si on le souhaite, en ses sels d'addition à un acide ou une base pharmaceutiquement acceptable.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 10 à 1000 mg par jour en une ou plusieurs prises.

Les exemples suivants illustrent l'invention mais ne la limitent en aucune façon.

Les produits de départ utilisés sont des produits de départ connus ou préparés selon des modes opératoires connus.

Les composés décrits dans les préparations conduisent à des intermédiaires de synthèse utiles pour la préparation des composés de l'invention.

### PREPARATION A : 3-[6-(4-Chlorophénylsulfonyl)amino-2-vinyl-5,6,7,8-tétrahydronapht-1-yl] propanoate de méthyle

### Stade A : 6-(4-Chlorophénylsulfonyl)amino-2-triméthylsilyl-5,6,7,8-tétrahydronapht-1-ylcarboxylate d'éthyle

290 mmoles de 6-(4-chlorophénylsulfonyl)amino-2-oxo-5,6,7,8-tétrahydro-2H-benzo[e] pyrane (composé 1) sont ajoutées à 540 g de triméthylsilyl propionate d'éthyle. Le mélange réactionnel est porté à 200°C en agitant. Après homogénéisation, 290 mmoles du composé 1 sont ajoutées et le mélange réactionnel est agité à 200°C jusqu'à une nouvelle homogénéisation. 290 mmoles du composé 1 sont alors ajoutés à nouveau et le mélange réactionnel est agité à 200°C pendant 16 heures. Le triméthylsilyl propionate d'éthyle en excès est distillé. Le résidu est purifié par une chromatographie sur colonne de silice en utilisant comme éluant un mélange cyclohexane/acétate d'éthyle (80/20), et le produit attendu est obtenu après cristallisation dans l'éther.
**Point de fusion : 104°C**

### Stade B : 6-(4-Chlorophénylsulfonyl)amino-2-triméthylsilyl-1-hydroxyméthyl-5,6,7,8-tétrahydronaphtalène

645 mmoles d'hydrure de lithium aluminium sont placées sous agitation dans 350 ml d'éther anhydre. Une solution de 28,7 g de chlorure d'aluminium dans 700 ml d'éther anhydre est ajoutée à température ambiante, suivie d'une solution de 215 mmoles du composé décrit au stade précédent. L'ensemble est agité une nuit à température ambiante. Après addition de 100 ml de méthanol et filtration sur célite des sels d'aluminium, on obtient le produit attendu.

### Stade C : 6-(4-Chlorophénylsulfonyl)amino-2-triméthylsylyl-5,6,7,8-tétrahydronapht-1-yl-carboxaldéhyde

A une solution de 90 g du produit obtenu au stade précédent dans 1 1 de dichlorométhane est ajouté à température ambiante 239 g de dichromate de 4-benzylpyridinium. Après agitation 1 heure à température ambiante, le solvant est évaporé, de l'acétate d'éthyle est ajouté. Les sels de chrome précipitent et sont filtrés. Le filtrat est lavé à l'acide chlorhydrique 0,1 N, séché. Après purification par chromatographie sur colonne de silice en utilisant comme éluant un mélange cyclohexane/acétate d'éthyl (80/20), on obtient le produit attendu.

### Stade D : 6-(4-Chlorophénylsulfonyl)amino-2-iodo-5,6,7,8-tétrahydronapht-1-yl-carboxaldéhyde

A une solution de 158 mmoles du produit obtenu au stade précédent dans 650 ml de dichlorométhane est ajouté à température ambiante 250 ml d'une solution 1M de chlorure d'iode dans le dichlorométhane. Après agitation une nuit à température ambiante, on évapore la majeure partie du solvant, on ajoute de l'éther, ce qui provoque la précipitation du produit attendu.

### Stade E : 6-(4-Chlorophénysulfonyl)amino-2-vinyl-5,6,7,8-tétrahydronapht-1-yl-carboxaldéhyde

150 mmoles du produit obtenu au stade précédent, 50 g de vinyltributylétain, 5 g de tétrakis (triphénylphosphine) palladium, sont portés sous agitation à 110°C, 3 heures dans 300 ml de N-méthyl-pyrrolidinone. Le solvant est évaporé, le résidu est repris dans 200 ml de dichlorométhane et traité par une solution aqueuse 10 % de fluorure de potassium. Après extraction, séchage et purification par une chromatographie sur colonne de silice en utilisant comme éluant un mélange acétate d'éthyle/cyclohexane (80/20), 46.2 g du produit attendu est obtenu.

### Stade F : 3-[6-(4-Chlorophénylsulfonyl)amino-2-vinyl-5,6,7,8-tétrahydronapht-1-yl]prop-2-ènoate de méthyle

123 mmoles du composé obtenu au stade précédent et 50 g de (carbométhoxyméthylidène)triphénylphosphorane sont portées au reflux 3 heures dans 300 ml de toluène. Après évaporation du solvant et purification du résidu par chromatographie sur colonne de silice en utilisant comme éluant un mélange cyclohexane/acétate d'éthyle (50/50), on obtient le produit attendu.

### Stade G : 3-[6-(4-Chlorophénylsulfonyl)amino-2-vinyl-5,6,7,8-tétrahydronapht-1-yl] propanoate de méthyle

A 2 400 ml d'une solution 0,1 N d'iodure de samarium dans le THF, placés sous agitation, est ajoutée une solution de 123 mmoles du composé obtenu au stade précédent. L'ensemble est agité 1 heure à température ambiante. Après addition de 150 ml de méthanol anhydre. le mélange est agité une nuit. Le solvant est alors évaporé, le résidu repris dans le dichlorométhane et lavé à l'acide chlorhydrique 0,1 N. Les phases organiques sont séchées et après purification par chromatographie sur colonne en utilisant comme éluant un mélange cyclohexane/acétate d'éthyle (80/20), on obtient le produit attendu après cristallisation dans l'éther.
**Point de fosion : 138°C**

### PREPARATION B : 3-[6-(4-Chlorophénylsulfonyl)amino-3-vinyl-5,6,7,8-tétrahydronapht-1-yl] propanoate de méthyle

### Stade A : 3-Bromo-6-(4-chlorophénylsulfonyl)amino-2-triméthylsilyl-5,6,7,8-tétrahydronapht-1-yl-carboxylate d'éthyle

Le produit attendu est obtenu selon le procédé décrit au stade A de la préparation A en utilisant la 3-bromo-6-(4-chlorophénylsulfonyl)amino-2-oxo-5,6,7,8-tétrahydro-2H benzo [e]pyrane comme produit de départ.
**Point de fusion : 134°C**

### Stade B : 3-Bromo-6-(4 -chlorophénylsulfonyl)amino-5,6,7,8-tétrahydronapht-1-yl-carboxylate d'éthyle

73 mmoles du composé décrit au stade précédent sont portées au reflux 4 heures dans 300 ml d'acide trifluoroacétique. Après évaporation de l'acide, le résidu est purifié par chromatographie sur colonne en utilisant comme éluant un mélange cyclohexane/acétate d'éthyle (80/20) et conduit au produit attendu.

### Stade C : 3-Bromo-6-(chlorophénylsulfonyl)amino-1-hydroxyméthyl-5,6,7,8-tétrahydronaphtalène

Le produit attendu est obtenu selon le procédé décrit au stade B de la préparation A à partir du composé obtenu au stade précédent.

### Stade D : 3-Bromo-6-(chlorophénylsulfonyl)amino-5,6,7,8-tétrahydronapht-1-yl, carboxaldéhyde

Le produit attendu est obtenu selon le procédé décrit au stade C de la préparation A à partir du composé décrit au stade précédent.
**Point de fusion : 145°C**

### Stade E : 3-[3-Bromo-6-(4-chlorophénylsulfonyl)amino-5,6,7,8-tétrahydronapht-1-yl] prop-2-ènoate de méthyle

A une solution de 30 mmoles du produit attendu au stade précédent dans 200 ml de toluène, est rajouté 12 g de (carbométhoxyméthylidène)triphénylphosphorane. La solution est portée au reflux 3 heures. Après évaporation du solvant et purification du résidu par chromatographie sur colonne de silice en utilisant comme éluant un mélange cyclohexane/acétate d'éthyle (50/50), on obtient le produit attendu.

### Stade F : 3-[3-Bromo-6-(4-chlorophénylsulfonyl)amino-5,6,7,8-tétrahydronaphyl-1-yl] propanoate de méthyle

A une solution de 35 mmoles du produit obtenu au stade précédent dans 100 ml de méthanol sont ajoutés 2,10 g d'hexahydrate de chlorure de cobalt puis par portion 2,66 g de borohydrure de sodium. Le mélange réactionnel est agité 2 heures à température ambiante puis filtré. Le solvant est évaporé. Le résidu est purifié par une chromatographie sur colonne de silice en utilisant comme éluant un mélange cyclohexane/acétate d'éthyle (80/20) et conduit au produit attendu après cristallisation dans l'éther.
**Point de fusion : 110°C**

### Stade G : 3-[6-(4-Chlorophénylsulfonyl)amino-3-vinyl-5,6,7,8-tétrahydronapht-1-yl] propanoate de méthyle

Le produit attendu est obtenu selon le procédé décrit au stade E de la préparation A à partir du composé décrit au stade précédent.

### PREPARATION C : 3-{6-[(4-Chlorophénylsulfonyl)amino]-3-vinyl-5,6,7,8-tétrahydronapht-1-yl}propanoate de tert-butyle

Le produit attendu est obtenu selon le procédé décrit dans la préparation B en remplaçant au stade E le (carbométhoxyméthylidène)triphénylphosphorane par le (carbo-tert-butoxyméthylidène)triphénylphosphorane.

### PREPARATION D : 3-{6-[(4-Chlorophénylsulfonyl)amino]-2-(2-hydroxyéthyl)-5,6,7,8-tétrahydronapht-1-yl}prop-2-ènoate de méthyle

### Stade A : {6-[(4-Chlorophénylsulfonyl)amino]-2-(2-hydroxyéthyl)-5,6,7,8-tétrahydronapht-1-yl}carboxaldéhyde

A 7,6 mmoles du composé décrit dans la préparation A, stade E, dans 35 ml de THF sont ajoutés 20 ml d'une solution 1M de complexe borane-tétrahydrofurane dans le THF. Après 2 heures d'agitation à température ambiante, la solution est refroidie à 0°C et 8,5 ml d'eau puis 7,6 g de tétrahydrate de perborate de sodium sont ajoutés.

Après agitation pendant 1 nuit, le THF est évaporé, et le résidu repris dans du dichlorométhane, puis extrait. La phase organique est séchée, concentrée et purifiée par chromatographie sur gel de silice en utilisant comme éluant un mélange acétate d'éthyle/cyclohexane (50/50), pour conduire au composé attendu.

### Stade B : 3-{6-[(4-Chlorophénylsulfonyl)amino]-2-(2-hydroxyéthyl)-5,6,7,8-tetrahydronapht-1-yl}prop-2-ènoate de méthyle

Le produit attendu est obtenu selon le procédé décrit dans la préparation A, stade F, à partir du composé décrit au stade précédent.

### PREPARATION E : 3-{6-[(4-Chlorophénylsulfonyl)amino]-3-(2-hydroxyéthyl)-5,6,7,8-tétrahydronapht-1-yl}prop-2-ènoate de méthyle

### Stade A : {6-[(4-Chlorophénylsulfonyl)amino]-3-vinyl-5,6,7,8-tétrahydronapht-1-yl} carboxaldéhyde

Le produit attendu est obtenu selon le procédé décrit dans la préparation A, stade E, à partir du composé décrit dans la préparation B, stade D.

### Stade B : {6-[(4-Chlorophénylsulfonyl)amino]-3-(2-hydroxyéthyl)-5,6,7,8-tétrahydronapht-1-yl}carboxaldéhyde

Le produit attendu est obtenu selon le procédé décrit dans la préparation D, stade A, à partir du composé décrit au stade précédent.

### Stade C : 3-{6-[(4-Chlorophénylsulfonyl)amino]-3-(2-hydroxyéthyl)-5,6,7,8-tétrahydronapht-1-yl}prop-2-ènoate de méthyle

Le produit attendu est obtenu selon le procédé décrit dans la préparation A, stade F, à partir du composé décrit au stade précédent.

### PREPARATION F : 3-{3-Allyl-6-[(4-chlorophénylsulfonyl)amino]-5,6,7,8-tétrahydronapht-1-yl}propanoate de méthyle

Le produit attendu est obtenu selon le procédé décrit dans la préparation B, en remplaçant, au stade G le vinyltributylétain par l'allyltributylétain.

### PREPARATION G : 3-{6-[(4-Chlorophénylsulfonyl)amino]-3-vinyl-5,6,7,8-tétrahydronapht-1-yl}carboxylate d'éthyle

Le produit attendu est obtenu selon le procédé décrit dans la préparation A, stade E, à partir du composé décrit dans la préparation B, stade B.

### PREPARATION H : 3-{6-[(4-Chlorophénylsulfonyl)amino]-2-(2-hydroxyéthoxy)-5,6,7,8-tétrahydronapht-1-yl}propanoate de méthyle

### Stade A : {6-[(4-Chlorophénylsulfonyl)amino]-2-hydroxy-5,6,7,8-tétrahydronapht-1-yl} carboxaldéhyde

Le produit attendu est obtenu selon le procédé décrit par Vollhart (Angew Chem. Int. Eng., 1984, p.539) à partir du composé décrit dans la préparation A, stade C.

### Stade B : {6-[(4-Chlorophénylsulfonyl)amino]-2-(2-hydroxyéthoxy)-5,6,7,8-tétrahydronapht-1-yl}carboxaldéhyde

Une solution de 2 mmoles de composé décrit au stade précédent dans 25 ml d'acétone est chauffée à reflux en présence de 3,8 mmoles de carbonate de potassium pendant 10 minutes. Puis 3,8 mmoles de 2-bromoéthanol sont ajoutées et le milieu est chauffé à reflux pendant 6 heures. Après refroidissement et filtration, le filtrat est concentré. Le résidu obtenu est repris dans un mélange acétate d'éthyle/eau et extrait. La phase organique est lavée par une solution de soude à 20 % puis par une solution aqueuse saturée de chlorure de sodium, séchée et concentrée pour conduire au composé attendu

### Stade C : 3-{6-[(4-Chlorophénylsulfonyl)amino]-2-(2-hydroxyéthoxy)-5,6,7,8-tétrahydronapht-1-yl}propanoate de méthyle

Le produit attendu est obtenu selon le procédé décrit dans la préparation A, stades F et G, à partir du composé décrit dans le stade précédent.

### PREPARATION 1 : 3-{6-[(4-Chlorophénylsulfonyl)amino]-2,3-bisvinyl-5,6,7,8-tétrahydronapht-1-yl}propanoate de méthyle

### Stade A : 3-{3-Bromo-6-[(4-chlorophénylsulfonyl)amino]-2-iodo-5,6,7,8-tétrahydronapht-1-yl}carboxylate d'éthyle

Le produit attendu est obtenu selon le procédé décrit dans la préparation A, stade D à partir du composé décrit dans la préparation B, stade A.

### Stade B : {3-Bromo-6[(4-chlorophénylsulfonyl)amino]-2-iodo-5,6,7,8-tétrahydronapt-1-yl}carboxaldéhyde

Le produit attendu est obtenu selon le procédé décrit dans la préparation A, stades B et C, à partir du composé décrit au stade précédent.

### Stade C : 3-{3-Bromo-6-[(4-chlorophénylsulfonyl)amino]-2-iodo-5,6,7,8-tétrahydronapht-1-yl}propanoate de méthyle

Le produit attendu est obtenu selon le procédé décrit dans la préparation A, stades F et G, à partir du composé décrit au stade précédent.

### Stade D : 3-{6-[(4-Chlorophénylsulfonyl)amino]-2,3,-bisvinyl-5,6,7,8-tétrahydronapht-1-yl} propanoate de méthyle

Le produit attendu est obtenu selon le procédé décrit dans la préparation A, stade E, à partir du composé décrit au stade précédent, en respectant la stoechiométrie.

### PREPARATION J : 3-{6-[(4-Chlorophénylsulfonyl)amino]-2-vinyl-5,6,7,8-tétrahydronapht-1-yl}propanoate de vinyl

Le produit attendu est obtenu par une réaction de transestérification à l'aide de l'alcool vinylique (préparé in situ par la méthode décrite par Rodler et al. J. Am. Chem. Soc., 1984, 106, 4029) du composé décrit dans la préparation B.

### PREPARATION K : 3-{6-[(4-Chlorophénylsulfonyl)aminoéthyl]-2-vinyl-5,6,7,8-tétrahydronapht-1-yl}propanoate de méthyle

Le produit attendu est obtenu selon le procédé décrit dans la préparation A à partir du 6-(4-chlorophénylsulfonyl)aminoéthyl-2-oxo-5,6,7,8-tétrahydro-2H-benzo[*e*]pyrane.

### PREPARATION L : 3-{6-[(4-Fluorophénylsulfonyl)amino]-2-vinyl-5,6,7,8-tétrahydronapht-1-yl}propanoate de méthyle

Le produit attendu est obtenu selon le procédé décrit dans la préparation A à partir du 6-(4-fluorophénylsulfonyl)amino-2-oxo-5,6,7,8-tétrahydro-2H-benzo[*e*]pyrane.

### PREPARATION M : 3-(6-Toluylsulfonylamino-2-vinyl-5,6,7,8-tétrahydronapht-1-yl) propanoate de méthyle

Le produit attendu est obtenu selon le procédé décrit dans la préparation A à partir du 6-toluylsulfonylamino-2-oxo-5,6,7,8-tétrahydro-2H-benzo[*e*]pyrane.

### PREPARATION N : 3-{5-{2-[(4-Chlorophénylsulfonyl)amino]éthyl}-2-vinyl-phényl} propanoate de méthyle

### PREPARATION O : 3-{5-{2-[(4-Chlorophénylsulfonyl)amino]éthyl}-3-vinyl-phényl} propanoate de méthyle

### EXEMPLE 1 : 3-{[6-(4-Chlorophénylsulfonyl)amino]-2-(2-nitroxyéthyl)-5,6,7,8-tétrahydronapht-1-yl)]}propanoate de méthyle

### Stade A : 3-[6(4-Chlorophénylsulfonyl)amino-2-(2-hydroxyéthyl)-5,6,7,8, tétrahydronapht-1-yl)]propanoate de méthyle

A 11,5 mmoles du composé décrit dans la préparation A dans 50 ml de THF sont ajoutés 25 ml d'une solution 1M de complexe borane-tétrahydrofurane dans le THF. Après agitation 2 heures à température ambiante, la solution est refroidie à 0°C et sont ajoutés 12,5 ml d'eau distillé, puis 11,5 g de tétrahydrate de perborate de sodium. Après agitation la nuit, le THF est évaporé. Après extraction au dichlorométhane, séchage et évaporation du solvant, le résidu est purifié par chromatographie sur colonne de silice en utilisant comme éluant un mélange acétate d'éthyle/cyclohexane (50/50), et conduit au produit attendu.
**Point de fusion : 132°C**

### Stade B : 3-[6-(4-Chlorophénylsulfonyl)amino-2-(2-tosyloxyéthyl)-5,6,7,8-tétrahydronapht-1-yl)]propanoate de méthyle

A 2,41 mmoles du composé obtenu au stade précédent dans 20 ml de dichlorométhane, sont ajoutées 0,8 ml de pyridine puis 0,92 g de chlorure de tosyle. Après agitation à température ambiante une nuit, le mélange est lavé à l'acide chlorhydrique IN et séché. Après évaporation du solvant et purification par chromatographie sur colonne de silice en utilisant comme éluant un mélange cyclohexane/acétate d'éthyle (70/30), on obtient le produit attendu.

### Stade C : 3-[6-(4-Chlorophénylsulfonyl)amino-2-(2-nitroxyéthyl)-5,6,7,8-tétrahydronapht-1-yl]propanoate de méthyle

A 10,4 mmoles du composé décrit au stade précédent et 0,47 g de nitrate de tétrabutylammonium sont portés au reflux 2 heures dans 50 ml de toluène. Après évaporation du toluène et purification par une chromatographie sur colonne de silice en utilisant comme éluant un mélange cyclohexane/acétate d'éthyle (70/30), on obtient le produit attendu.

### EXEMPLE 2 : Acide 3-{6-[(4-chlorophénylsulfonyl)amino]-2-(2-nitroxyéthyl)-5,6,7,8-tétrahydronapht-1-yl}propionique

7,97 mmoles du composé décrit dans l'exemple 1 sont portées au reflux 2 heures dans 20 ml de méthanol en présence de 1,6 ml de soude IN. La solution est filtrée et la majeure partie du méthanol est évaporé, 10 ml d'eau sont ajoutés, puis de l'acide chlorhydrique IN jusqu'à pH = 1. Le précipité obtenu est séché et recristallisé dans l'éther et conduit au composé attendu.
**Point de fusion : 175°C (dec)**

### EXEMPLE 3 : 3-[6-(4-Chlorophénylsulfonyl)amino-2-nitroxyméthyl-5,6,7,8-tétrahydronapht-1-yl]propanoate de méthyle

### Stade A : 3-[6-(4-Chlorophénylsulfonyl)amino-2-hydroxyméthyl-5,6,7,8-tëtrahydronapht-1-yl]propanoate de méthyle

2 g du produit décrit dans la préparation A sont soumis à ozonolise à -78°C dans 150 ml de dichlorométhane. Après 2 heures à -78°C, 150 ml de méthanol sont ajoutés puis lentement 5 g de borohydrure de sodium. Le mélange réactionnel est amené à température ambiante et agité une nuit. Après ajout d'une solution aqueuse saturée de bicarbonate de sodium, évaporation de la majeure partie du méthanol, le mélange réactionnel est extrait au dichlorométhane. La phase organique est séchée et évaporée. Une purification par chromatographie sur colonne de silice, en utilisant comme éluant un mélange acétate d'éthyle/cyclohexane (50/50), conduit au produit attendu.

### Stade B : 3-[6-(4-Chlorophénylsulfonyl)amino-2-bromométhyl-5,6,7,8-tétrahydronapht-1-yl]propanoate de méthyle

A une solution de 2,68 g de triphénylphosphine dans 50 ml de dichlorométhane est ajoutée à température ambiante une solution de 2,12 g de tétrabromocarbone dans 25 ml de dichlorométhane. La solution jaune est agitée 15 minutes et une solution de 1,77 g du produit décrit au stade précédent dans 50 ml de dichlorométhane est ajoutée. Après agitation à température ambiante 1 heure, le solvant est évaporé. Une purification par chromatographie sur colonne de silice en utilisant comme éluant un mélange cyclohexane/acétate d'éthyle (80/20) conduit au produit attendu.

### Stade C : 3-[6-(4-Chlorophénylsulfonyl)amino-2-nitroxyméthyl-5,6,7,8-tétrahydronapht-1-yl]propanoate de méthyle

A une solution de 1 mmole du produit obtenu au stade précédent dans 10 ml d'acétonitrile est ajoutée une solution de 250 mg de nitrate d'argent dans 10 ml d'acétonitrile. Après agitation à température ambiante une nuit, le précipité de bromure d'argent est filtré. Le solvant est évaporé et une purification par chromatographie sur colonne de silice en utilisant comme éluant un mélange cyclohexane/acétate d'éthyle (80/20) conduit au produit attendu.

### EXEMPLE 4 : Acide 3-[6-(4-chlorophénylsulfonyl)amino-2-nitroxyméthyl-5,6,7,8-tétrahydro-napht-1-yl]propionique

Le produit attendu est obtenu selon le procédé décrit dans l'exemple 2, à partir du composé obtenu dans l'exemple 3.

### EXEMPLE 5 : 3-{6-[(4-Chlorophénylsulfonyl)amino]-3-(2-nitroxyéthyl)-5,6,7,8-tétrahydronapht-1-yl}propanoate de méthyle

### Stade A : 3-{6-[(4-Chlorophénylsulfonyl)amino]-3-(2-hydroxyéthyl)-5,6,7,8-tétrahydronapht-1-yl}propanoate de méthyle

Le produit attendu est obtenu selon le procédé décrit au stade A de l'exemple 1 à partir du composé décrit dans la préparation B.

### Stade B : 3-{6-[(4-Chlorophénylsulfonyl)amino]-3-(2-tosyloxyéthyl)-5,6,7,8-tétrahydronapht-1-yl}propanoate de méthyle

Le produit attendu est obtenu selon le procédé décrit au stade B de l'exemple 1 à partir du composé obtenu au stade précédent.

### Stade C : 3-6-(4-Chlorophénylsulfonyl)amino-3-(2-nitroxyéthyl)-5,6,7,8-tétrahydronapht-1-yl}propanoate de méthyle

Le produit attendu est obtenu selon le procédé décrit au stade C de l'exemple 1 à partir du composé obtenu au stade précédent.

### EXEMPLE 6 : Acide 3-{6-[(4-chlorophénylsulfonyl)amino-3-(2-nitroxyéthyl)-5,6,7,8-tétrahydronapht-1-yl}propionique

Le produit attendu est obtenu selon le procédé décrit dans l'exemple 2 à partir du composé obtenu dans l'exemple 5.

### EXEMPLE 7 : 3-{6-[(4-Chlorophénylsulfonyl)amino]-2-(2-nitrosothio-2-méthylpropylcarbamoyl)-5,6,7,8-tétrahydronapht-1-yl}propanoate de méthyle

### Stade A : 3-[6-(4-Chlorophénylsulfonyl)amino-2-formyl-5,6,7,8-tétrahydronapht-1-yl)] propanoate de méthyle

A une solution de 23 mmoles du produit décrit dans la préparation A, dans un mélange de 100 ml de dioxane et 50 ml d'eau, est ajouté à température ambiante 2,5 g d'une solution de tétroxyde d'osmium dans le 2-méthyl-2-propanol, puis 20 g de périodate de sodium. Après agitation une nuit à température ambiante, la suspension est filtrée, le filtrat est évaporé. L'huile obtenue est reprise dans le dichlorométhane, cette phase organique est lavée à l'eau, séchée et évaporée. Le résidu est purifié par chromatographie sur colonne de silice en utilisant comme éluant un mélange cyclohexane/acétate d'éthyle (60/40) et conduit au produit attendu.
**Point de fusion : 110°C**

### Stade B : Acide 6-(4-chlorophénylsulfonyl)amino-1-(2-méthoxycarbonyl-éthyl)-5,6,7,8-tétrahydronaphtalène-2-carboxylique

A une solution de 6,88 mmoles du produit obtenu au stade précédent dans 30 ml d'acétone est ajoutée à température ambiante une solution de 2,7 g de permanganate de potassium dans 50 ml d'un mélange acétone/eau (50/50). La solution est agitée 2 heures à température ambiante. Après filtration, 100 ml de méthanol est ajouté et la solution est portée au reflux 2 heures. Après filtration, évaporation du solvant, 100 ml d'eau est ajoutée. Après acidification à pH = 2, extraction au dichlorométhane, séchage et évaporation du solvant, une purification du résidu par chromatographie sur colonne de silice en utilisant comme éluant un mélange dichlorométhane/méthanol (95/5) et conduit au produit attendu.

### Stade C : 3-[6-(4-Chlorophénylsulfonyl)amino]-2-(2-méthyl-propylcarbamoyl)-5,6,7,8-tétrahydronapht-1-yl]propanoate de méthyle

Le produit attendu est obtenu selon le procédé décrit dans J. Org. Chem., 59, 7019-7026, (1994). A une solution de 4,97 mmoles du produit décrit au stade précédent dans 50 ml de benzène est ajouté 5 ml de chlorure de thionyle. Après 1 heure de reflux, la solution est évaporée à sec pour donner le chlorure d'acide correspondant. A une solution de 0,75 g de chlorure de 1-amino-2-méthyl-2-propanethiol dans 50 ml d'acétonitrile sont ajoutés à température ambiante 3 ml de N-N-diisopropyléthylamine, puis une solution du chlorure d'acide précédent dans 50 ml d'acétonitrile. Après agitation 30 minutes à température ambiante, hydrolyse, extraction au dichlorométhane, séchage et évaporation du solvant, le résidu est purifié à l'aide d'une chromatographie sur colonne de silice en utilisant comme éluant un mélange acétate d'éthyle/cyclohexane (30/70) pour conduire au produit attendu.

### Stade D : 3-{6-[(4-Chlorophénylsulfonyl)amino]-2-(2-nitrosothio-2-méthylpropylcarbamoyl)-5,6,7,8-tétrahydronapht-1-yl}propanoate de méthyle

A une solution de 1,06 g du produit obtenu au stade précédent dans 25 ml de dichlorométhane est ajouté 0,3 g de nitrite de terbutyle à température ambiante. Après agitation 15 minutes à température ambiante, l'évaporation du solvant conduit au produit attendu.

### EXEMPLE 8 : Acide 3-{6-[(4-chlorophénylsulfonyl)amino]-2-(2-nitrosothio-2-méthylpropylcarbamoyl)-5,6,7,8-tétrahydronapht-1-yl}propionique

Le produit attendu est obtenu selon le procédé décrit dans l'exemple 2 à partir du composé décrit dans l'exemple 7.

### EXEMPLE 9 : 3-{6-[(4-Chlorophénylsulfonyl)amino]-3-nitroxyméthyl-5,6,7,8-tétrahydronapht-1-yl}propanoate de méthyle

Le produit attendu est obtenu selon le procédé décrit dans l'exemple 3 à partir du composé décrit dans la préparation B.

### EXEMPLE 10 : 3-{6-[(4-Chlorophénylsulfonyl)amino]-3-nitroxyméthyl-5,6,7,8-tétrahydronapht-1-yl}propanoate de tert-butyle

### Stade A : 3-{6-[(4-Chlorophénylsulfonyl)amino]-3-formyl-5,6,7,8-tétrahydronapht-1-yl}propanoate de tert-butyle

Le produit attendu est obtenu selon le procédé décrit dans l'exemple 7, stade A, à partir du composé décrit dans la préparation C.

### Stade B : 3-{-[(4-Chlorophénylsulfonyl)amino]-3-hydroxyméthyl-5,6,7,8-tétrahydronapht-1-yl}propanoate de tert-butyle

A une solution de 6,5 mmoles (3 g) du produit au stade précédent dans 50 ml de méthanol sont ajoutées à température ambiante 26,4 mmoles (1 g) de borohydrure de sodium. Le mélange réactionnel est agité 30 minutes à température ambiante. Après ajout d'une solution aqueuse saturée de bicarbonate de sodium, évaporation de la majeure partie du méthanol, le mélange réactionnel est extrait au dichlorométhane. La phase organique est séchée et évaporée. Une purification par chromatographie par colonne de silice, en utilisant comme éluant un mélange acétate d'éthyle/cyclohexane (50/50), conduit au produit attendu.

### Stade C : 3-{6-[(4-Chlorophénylsulfonyl)amino]-3-bromométhyl-5,6,7,8-tétrahydronapht-1-yl}propanoate de tert-butyle

Le produit attendu est obtenu selon le procédé décrit dans l'exemple 3, stade B, à partir du composé décrit au stade précédent.

### Stade D : 3-{6-[(4-Chlorophénylsulfonyl)amino]-3-nitroxyméthyl-5,6,7,8-tétrahydronapht-1-yl}propanoate de tert-butyle

Le produit attendu est obtenu selon le procédé décrit dans l'exemple 3, stade C, à partir du composé décrit au stade précédent.

### EXEMPLE 11 : Acide 3-{6-[(4-Chlorophénylsulfonyl)amino]-3-nitroxyméthyl-5,6,7,8-tétrahydronapht-1-yl}propionique

### Méthode A :

Le produit attendu est obtenu selon le procédé décrit dans l'exemple 2, stade A, à partir du composé décrit dans l'exemple 9.

### Méthode B :

On fait buller du chlorure d'hydrogène gazeux dans une solution de 0,1 g de composé décrit dans l'exemple 10 dans 20 ml d'éther anhydre. L'évaporation du solvant conduit au produit attendu.

### EXEMPLE 12 : 3-{6-[(4-Chlorophénylsulfonyl)amino]-2-(2-nitroxyéthyl)-5,6,7,8-tétrahydronapht-1-yl}prop-2-énoate de méthyle

### Stade A : 3-{6-[(4-Chlorophénylsulfonyl)amino]-2-(2-tosyloxyéthyl)-5,6,7,8-tétrahydronaphl-1-yl)prop-2-énoate de méthyle

Le produit attendu est obtenu selon le procédé décrit dans l'exemple 1, stade B, à partir du composé décrit dans la préparation D.

### Stade B : 3-{6-[(4-Chlorophénylsulfonyl)amino]-2-(2-nitroxyéthyl)-5,6,7,8-tétrahydronapht-1-yl}prop-2-énoate de méthyle

Le produit attendu est obtenu selon le procédé décrit dans l'exemple 1, stade C, à partir du composé décrit au stade précédent.

### EXEMPLE 13 : Acide 3-{6-[(4-chlorophénylsulfonyl)amino]-2-(2-nitroxyéthyl)-5,6,7,8-tétrahydronapht-1-yl}prop-2-énoïque

Le produit attendu est obtenu selon le procédé décrit dans l'exemple 2, stade B, à partir du composé décrit dans l'exemple 12.

### EXEMPLE 14 : 3-{6-[(4-Chlorophénylsulfonyl)amino]-2-(2-nitrosooxyéthyl)-5,6,7,8-tétrahydronapht-1-yl}propanoate de méthyle

Le produit attendu est obtenu selon le procédé décrit dans l'exemple 1, stade C, en utilisant comme produit de départ le composé décrit dans l'exemple 1, stade A, et en remplaçant le nitrate de tétrabutylammonium par le nitrite de tert-butyle.

### EXEMPLE 15 : Acide 3-{6-[(4-chlorophénylsulfonyl)amino]-2-(2-nitrosooxyéthyl)-5,6,7, 8-tétrahydronapht-1-yl}propionique

Le produit attendu est obtenu selon le procédé décrit dans l'exemple 2 à partir du composé décrit dans l'exemple 14.

### EXEMPLE 16 : 3-{6-[(4-Chlorophénylsulfonyl)amino]-3-(2-nitroxyéthyl)-5,6,7,8-tétrahydronapht-1-yl}prop-2-énoate de méthyle

Le produit attendu est obtenu selon le procédé décrit dans l'exemple 12 à partir du composé décrit dans la préparation E.

### EXEMPLE 17 : Acide 3-{6-[(4-chlorophénylsulfonyl)amino]-3-(2-nitrosooxyéthyl)-5,6,7, 8-tétrahydronapht-1-yl}prop-2-énoïque

Le produit attendu est obtenu selon le procédé décrit dans l'exemple 2 à partir du composé décrit dans l'exemple 16.

### EXEMPLE 18 : 3-{6-[(4-Chlorophénylsulfonyl)amino]-3-(3-nitroxypropyl)-5,6,7,8-tétrahydronapht-1-yl}propanotate de méthyle

Le produit attendu est obtenu selon le procédé décrit dans l'exemple 5 à partir du composé décrit dans la préparation F.

### EXEMPLE 19 : Acide 3-{6-[(4-chlorophénylsulfonyl)amino]-3-(3-nitroxypropyl)-5,6,7, 8-tétrahydronapht-1-yl}propionique

Le produit attendu est obtenu selon le procédé décrit dans l'exemple 2 à partir du composé décrit dans l'exemple 18.

### EXEMPLE 20 : 3-{6-[(4-Chlorophénylsulfonyl)amino]-3-(2-nitroxyéthyl)-5,6,7,8-tétrahydronapht-1-yl}carboxylate d'éthyle

Le produit attendu est obtenu selon le procédé décrit dans l'exemple 1 à partir du composé décrit dans la préparation G.

### EXEMPLE 21 : Acide 3-{6-[(4-chlorophénylsulfonyl)amino]-3-(2-nitroxyéthyl)-5,6,7,8-tétrahydronapht-1-yl}carboxylique

Le produit attendu est obtenu selon le procédé décrit dans l'exemple 2 à partir du composé décrit dans l'exemple 20.

### EXEMPLE 22 : 3-{6-[(4-Chlorophénylsulfonyl)amino]-2-(2-nitroxyéthoxy)-5,6,7,8-tétrahydronapht-1-yl}propanoate de méthyle

Le produit attendu est obtenu selon le procédé décrit dans l'exemple 1 à partir du composé décrit dans la préparation H.

### EXEMPLE 23 : Acide 3-{6-[(4-chlorophénylsulfonyl)amino]-2-(2-nitroxyéthoxy)-5,6,7,8-tétrahydronapht-1-yl}propionique

Le produit attendu est obtenu selon le procédé décrit dans l'exemple 2 à partir du composé décrit dans l'exemple 22.

### EXEMPLE 24 : 3-{6-[(4-Chlorophénylsulfonyl)amino]-2,3-bis-(2-nitroxyéthyl)-5,6,7,8-tétrahydronapht-1-yl}propanoate de méthyle

Le produit attendu est obtenu selon le procédé décrit dans l'exemple 1 à partir du composé décrit dans la préparation I, en respectant la stoechiométrie.

### EXEMPLE 25 : Acide 3-{6-[(4-chlorophénylsulfonyl)amino]-2,3-bis-(2-nitroxyéthyl)-5,6, 7,8-tétrahydronapht-1-yl}propionique

Le produit attendu est obtenu selon le procédé décrit dans l'exemple 2 à partir du composé décrit dans l'exemple 24.

### EXEMPLE 26 : 3-{6-[(4-Chlorophénylsulfonyl)amino]-2-(2-nitroxyéthyl)-5,6,7,8-tétrahydronapht-1-yl}propanoate de 2-nitroxyéthyle

Le produit attendu est obtenu selon le procédé décrit dans l'exemple 1 à partir du composé décrit dans la préparation J, en respectant la stoechiométrie.

### EXEMPLE 27 : 3-{6-[(4-Chlorophénylsulfonyl)amino]-2-(2-nitroxyéthyl)-5,6,7,8-tétrahydronapht-1-yl}propanoate de méthyle

Le produit attendu est obtenu selon le procédé décrit dans l'exemple 1 à partir du composé décrit dans la préparation K.

### EXEMPLE 28 : Acide 3-{6-[(4-chlorophénylsulfonyl)amino]-2-(2-nitroxyéthyl)-5,6,7,8-tétrahydronapht-1-yl}propionique

Le produit attendu est obtenu selon le procédé décrit dans l'exemple 2 à partir du composé décrit dans l'exemple 27.

### EXEMPLE 29 : 3-{6-[(4-Fluorophénylsulfonyl)amino]-2-(2-nitroxyéthyl)-5,6,7,8-tétrahydronapht-1-yl}propanoate de méthyle

Le produit attendu est obtenu selon le procédé décrit dans l'exemple 1 à partir du composé décrit dans la préparation L.

### EXEMPLE 30 : Acide 3-{6-[(4-fluorophénylsulfonyl)amino]-2-(2-nitroxyéthyl)-5,6,7,8-tétrahydronapht-1-yl}propionique

Le produit attendu est obtenu selon le procédé décrit dans l'exemple 2 à partir du composé décrit dans l'exemple 29.

### EXEMPLE 31 : 3-[6-Toluylsulfonylamino-2-(2-nitroxyéthyl)-5,6,7,8-tétrahydronapht-1-yl]propanoate de méthyle

Le produit attendu est obtenu selon le procédé décrit dans l'exemple 1 partir du composé décrit dans la préparation M.

### EXEMPLE 32 : Acide 3-[6-toluylsulfonylamino-2-(2-nitroxyéthyl)-5,6,7,8-tétrahydronapht-1-yl}propionique

Le produit attendu est obtenu selon le procédé décrit dans l'exemple 2 à partir du composé décrit dans l'exemple 31.

### EXEMPLE 33 : 3-{5-[2[(4-Chlorophénylsulfonyl)amino]éthyl]-2-(2-nitroxyéthyl)-5,6,7, 8-tétrahydronapht-1-yl}propanoate de méthyle

Le produit attendu est obtenu selon le procédé décrit dans l'exemple 1 partir du composé décrit dans la préparation N.

### EXEMPLE 34 : Acide 3-{5-[2-[(4-chlorophénylsulfonyl)amino]éthyl]-2-(2-nitroxyéthyl) phényl}propionique

Le produit attendu est obtenu selon le procédé décrit dans l'exemple 2 à partir du composé décrit dans l'exemple 33.

### EXEMPLE 35 : 3-{5-[2-[(4-Chlorophénylsulfonyl)amino]éthyl]-3-(2-nitroxyéthyl) phényl}propanoate de méthyle

Le produit attendu est obtenu selon le procédé décrit dans l'exemple 3 à partir du composé décrit dans la préparation O.

### EXEMPLE 36 : Acide 3-{5-[2-[(4-chlorophénylsulfonyl)amino]éthyl]-3-(2-nitroxyéthyl) phényl}propionique

Le produit attendu est obtenu selon le procédé décrit dans l'exemple 2 à partir du composé décrit dans l'exemple 35.

### Etude pharmacologique des dérivés de l'invention

### EXEMPLE 37 : Agrégation plaquettaire chez l'homme

Le sang veineux est obtenu de volontaires humains n'ayant pas pris de l'aspirine pendant au moins 14 jours précédent l'expérience. Le sang est prélevé sur citrate de sodium (0.109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. Les antagonistes sont testés suivant la procédure décrite dans l'exemple 19 en utilisant le U 46619 à la concentration de 0.3 µM. Les composés de l'invention inhibent l'agrégation plaquettaire induite par l'agoniste TXA₂, le U 46619. L'IC₅₀ des composés des exemples 2 et 4 sont égales à 140 nM. Cette valeur indique que les composés de l'invention sont des puissants antiagrégants plaquettaires agissant sur la voie TXA₂.

### EXEMPLE 38 : Détermination des valeurs pA₂ des antagonistes vis-à-vis du TXA₂ sur la veine saphène de lapin

Des lapins néo-zélandais (2.5 kg) ont été exsanguinés sous anesthésie (pentobarbital, 30 mg.kg) et les veines saphènes rapidement prélevées et découpées en anneaux de trois mm. Ces anneaux sont montés entre deux crochets dans des cuves thermostatées à 37°C contenant du liquide physiologique (composition en mM : NaCI 118 ; NaHCO₃ 25 ; Glucose 10 ; KCl 4.7 ; CaCl₂ 1.25 ; MgSO₄ 1.19 ; KHPO₄ 1.14).

La solution physiologique est bullée par un mélange de 95 % O₂ - 5 % CO₂. Le crochet inférieur constitue le point fixe tandis que le crochet supérieur est relié à un capteur de force isométrique. Les tissus sont mis sous tension de base de 1 gramme. Les substances pharmacologiques étudiées sont préparées immédiatement avant l'usage. Les drogues sont solubilitées dans l'eau ou dans le diméthylsulfoxyde.

Après le montage, les préparations sont laissées en repos pendant 90 minutes, des rinçages étant effectués toutes les 30 minutes. Après réajustement de la tension de base. une contraction par une dose unique de l'agoniste TXA₂, le U 46619 à 10⁻⁵M, est provoquée afin de régulariser les contractions suivantes. Après lavage et retour à la ligne de base. une première courbe effet/concentration est réalisée par adjonction de doses cumulatives d'U 46619 (10⁻⁹M à 10⁻⁵M ; l'espacement entre les doses est d'un demi-log). Cette première expérience permet de calculer la concentration efficace 50 % (EC₅₀) "témoin".

Cette EC₅₀ est calculée en routine de la manière suivante : les valeurs de tension sont d'abord converties en pourcentages par rapport à l'effet maximum, ces pourcentages sont ensuite reportés sur un graphique avec les pourcentages en ordonnée et les log (concentration) en abscisse. Une régression linaire est alors effectuée sur les points compris entre 10 % et 90 % (ce qui correspond à la partie linéaire de la courbe sigmoïde). La concentration correspondant au demi effet maximum (50 %) peut être facilement calculée à l'aide des paramètres de la droite. Après lavage et retour à la ligne de base, l'organe est mis en contact avec l'antagoniste (8 concentrations différentes pour chaque organe) pendant 20 minutes. Une deuxième courbe effet/concentration est alors réalisée en présence de l'antagoniste et l'EC₅₀ "traité" peut alors être calculée. On dispose ainsi de tous les éléments permettant le calcul du pA₂ (antagonisme compétitif) ou pD₂ (antagonisme non compétitif).

Le pA₂ (qui représente le logarithme négatif de la concentration d'antagoniste en présence de laquelle il faut 2 fois plus d'antagoniste pour obtenir le même effet) est déterminé en reportant sur une graphe les valeurs de log (L/l-1) par rapport à log (concentration d'antagoniste) avec L=effet en présence d'antagoniste et l=effet témoin.

Lors de ce test, les valeurs des pA₂ des composés de l'invention sont les suivantes :
Exemple 2: 9.5
Exemple 6: 9.8

Ces valeurs indiquent que les composés de l'invention sont de puissants antagonistes des TP-récepteurs vasculaires.

### EXEMPLE 39 Détermination des relaxations non-entothélium dépendantes dans la veine saphène de lapin

Les veines saphène de lapin sont préparées et montées comme décrites dans l'exemple précédent. Après stabilisation, une contraction au KCI (60 mM) est provoquée. Pendant cette contraction, des concentrations croissantes des substances de l'invention sont administrées et les relaxations qui en résultent sont notées permettant le calcul d'une concentration relaxante efficace 50 % (IC₅₀). Elle est calculée comme décrite dans l'exemple précédent.
Pour les substances de l'invention, des IC₅₀ suivantes ont été obtenues :
Exemple 2: 10 µM
Exemple 6: 7.7 µM

Ces valeurs indiquent que le NO, libéré par nos substances provoque la relaxation des veines. Cela a été confirmé en utilisant un inhibiteur sélectif de la guanylate cyclase, l'ODQ, qui est capable d'inhiber les relaxations démontrant qu'elles sont dues à la production de GMP cyclique, le médiateur des relaxations provoquées par le NO.

### EXEMPLE 40 Composition pharmaceutique

| Formule de préparation pour 1000 comprimés dosés à 100 mg : | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) 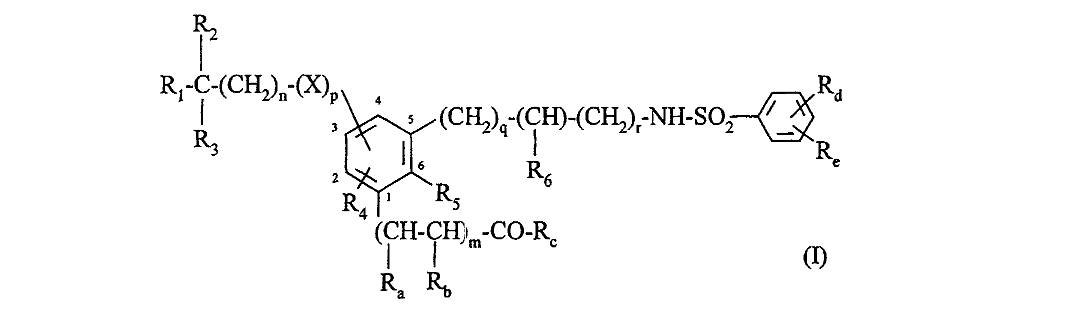 dans laquelle :
Rₐ, R_{b}, identiques ou différents, représentent un atome d'hydrogène ou un groupement alkyle (C₁-C₆) linéaire ou ramifié,
ou bien Rₐ, R_{b} forment ensemble une liaison,
R_{c} représente un groupement hydroxy, alkoxy (C₁-C₆) linéaire ou ramifié (substitué éventuellement par un groupement -O-NO₂, -O-NO ou -S-NO), un groupement amino (substitué éventuellement par un ou deux groupements, identiques ou différents, alkyle (C₁-C₆) linéaire ou ramifié, phényle éventuellement substitué),
R₁ représente un atome d'hydrogène ou un groupement -O-NO₂, -O-NO ou -S-NO,
R₂, R₃, identiques ou différents, représentent un atome d'hydrogène ou un groupement alkyle (C₁-C₆) linéaire ou ramifié, ou phényle éventuellement substitué,
X représente un atome d'oxygène ou un groupement -NH-CO-,
m représente 0 ou 1,
n représente un nombre entier tel que 0 ≤ n ≤ 6,
p représente 0 ou 1,
R₄ représente un atome d'hydrogène, un groupement alkyle (C₁-C₆) linéaire ou ramifié, (éventuellement substitué par un groupement hydroxy), phényle éventuellement substitué ou un groupement : 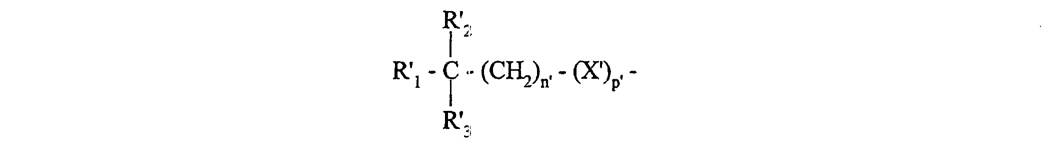 dans lequel :
R'₁ représente un atome d'hydrogène ou un groupement : -O-NO₂, -O-NO ou -S-NO,
R'₂, R'₃, identiques ou différents, représentent un atome d'hydrogène ou un groupement alkyle (C₁-C₆) linéaire ou ramifié, ou phényle éventuellement substitué,
X' représente un atome d'oxygène ou un groupement -NH-CO-,
n' représente un nombre entier tel que 0 ≤ n ≤ 6,
p' représente 0 ou 1,
R₅ représente un atome d'hydrogène ou un groupement alkyle (C₁-C₆), linéaire ou ramifié,
R₆ représente un atome d'hydrogène ou un groupement alkyle (C₁-C₆) linéaire ou ramifié, ou bien R₅ et R₆ forment ensemble une chaîne -(CH₂)ₜ- dans laquelle t représente 1 ou 2,
q représente 0,1 ou 2,
r représente un nombre entier tel que 0≤r≤6,
R_{d}, Rₑ, identiques ou différents, représentent un atome d'hydrogène, un atome d'halogène, un groupement alkyle (C₁-C₆) linéaire ou ramifié, un groupement alkoxy (C₁-C₆) linéaire ou ramifié, un groupement hydroxy ou un groupement trihalogénoalkyle (C₁-C₆) linéaire ramifié,
étant entendu qu'au moins un groupement -O-NO₂, -O-NO ou -S-NO soit présent en R₁, R₄ ou R_{c},
et que par phényle éventuellement substitué, on entend éventuellement substitué par un ou plusieurs, identiques ou différents, atomes d'halogène ou groupements alkyle (C₁-C₆) linéaire ou ramifié, alkoxy (C₁-C₆) linéaire ou ramifié, hydroxy ou trihalogénoalkyle (C₁-C₆) linéaire ou ramifié,
et leurs sels d'addition à un acide ou à une base pharmaceutiquement acceptable.

2. Composé de formule (I) selon la revendication 1 tels que R₁ représente -O-NO₂, -O-NO ou -S-NO.

3. Composé de formule (I) selon la revendication 1 tels que Rₐ et R_{b} représentent simultanément un atome d'hydrogène et m est égal à 1.

4. Composé de formule (I) selon la revendication 1 tels que R₅ et R₆ forment ensemble une chaîne -(CH₂)ₜ-.

5. Composé de formule (I) selon la revendication 1 tels que R₄ représente un atome d'hydrogène.

6. Composé de formule (I) selon la revendication 1 tels que p est égal à 0.

7. Composé de formule (I) selon la revendication 1 tels que R_{c} représente un groupement hydroxy ou un groupement alkoxy (C₁-C₆) linéaire ou ramifié.

8. Composé de formule (I) selon la revendication 1 tels que R_{d} représente un atome d'halogène tandis que Rₑ représente un atome d'hydrogène.

9. Composé de formule (I) selon la revendication 1 tels que R₁ représente un groupement -O-NO₂ tandis que p est égal à 0, Rₐ et R_{b} représentent chacun un atome d'hydrogène tandis que m est égal à 1, R₄ représente un atome d'hydrogène, R₅ et R₆ forment ensemble une chaîne -(CH₂)ₜ avec t égal à 2 tandis que q est égal à 1, et r est égal à 0, R_{d} représente un atome d'halogène tandis que Rₑ représente un atome d'hydrogène et R_{c} représente un groupement hydroxy ou un groupement alkoxy (C₁-C₆) linéaire ou ramifié.

10. Composé de formule (I) selon la revendication 1 qui est l'acide 3-{6-[(4-chlorophénylsulfonyl)amino]-2-(2-nitroxyéthyl)-5,6,7,8-tétrahydronapht-1-yl} propionique, ainsi que ses sels d'addition à une base pharmaceutiquement acceptable.

11. Composé de formule (I) selon la revendication 1 qui est l'acide 3-{6-[(4-chlorophénylsulfonyl)amino-3-(2-nitroxyéthyl)-5,6,7,8-tétrahydronapht-1-yl}propionique, ainsi que ses sels d'addition à une base pharmaceutiquement acceptable.

12. Composé de formule (I) selon la revendication 1 qui est l'acide 3-{6-[(4-chlorophénylsulfonyl)amino]-3-nitroxyméthyl-5,6,7,8-tétrahydronapht-1-yl}propionique, ainsi que ses sels d'addition à une base pharmaceutiquement acceptable.

13. Procédé de préparation des dérivés de formule (I) selon la revendication 1 tels que p=p'=0, **caractérisé en ce que** l'on utilise comme produit de départ un composé de formule (II): 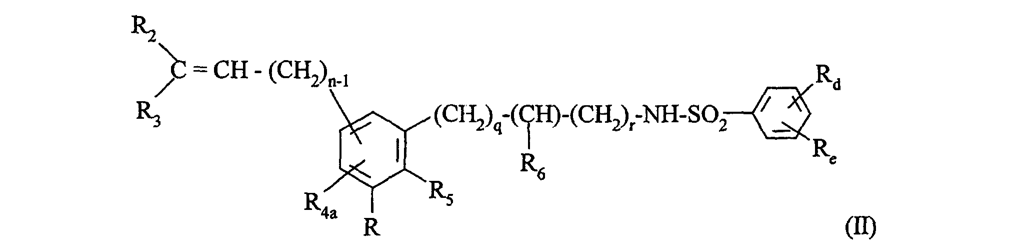 dans laquelle :
R_{d}, Rₑ, R₂, R₃, R₅, R₆, n, q, r ont la même signification que dans la formule (I),
R₄ₐ représente un atome d'hydrogène, un groupement alkyle (C₁-C₆) linéaire ou ramifié (éventuellement substitué par un groupement hydroxy), phényle éventuellement substitué ou un groupement 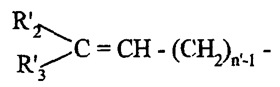 (dans lequel R'₂, R'₃ et n' ont la même signification que dans la formule (I)),
et R représente -CH₂-CH₂-CO₂R'_{c} ou -CO₂R'_{c} (dans lesquels R'_{c} représente un groupement alkyle (C₁-C₆) linéaire ou ramifié éventuellement substitué par un groupement hydroxy),
que l'on soumet à un traitement oxydant approprié de la double liaison en alcool correspondant,
pour conduire au composé de formule (III) : 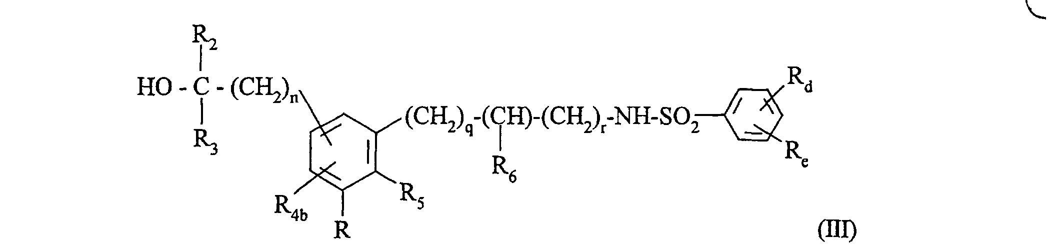 dans laquelle :
R, R_{d}, Rₑ, R₂, R₃, R₅, R₆, n, q et r ont la même définition que précédemment,
R_{4b} représente un atome d'hydrogène, un groupement alkyle (C₁-C₆) linéaire ou ramifié (éventuellement substitué par un groupement hydroxy), phényle éventuellement substitué ou un groupement 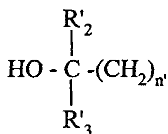 (dans lequel R'₂, R'₃ et n' ont la même signification que précédemment),
composé de formule (III) qui est éventuellement transformé lorsque R représente un groupement -CO₂Alk (Alk étant un groupement alkyle (C₁-C₆) linéaire ou ramifié) en aldéhyde correspondant puis qui subit une réaction de Wittig, pour conduire à un composé de formule (III) dans laquelle le groupement R est remplacé par le groupement -CH=CH-CO₂R'_{c}, R'_{c} étant tel que défini précédemment,
composé de formule (III):
**soit** dont on transforme le ou les groupements hydroxy en un groupement OTs (dans lequel Ts représente le groupement Tosyle) ou en un atome de brome et qui subit l'action du nitrate de tétrabutylammonium ou du nitrate d'argent,
pour conduire au composé de formule (I/a), cas particulier des composés de formule (I): 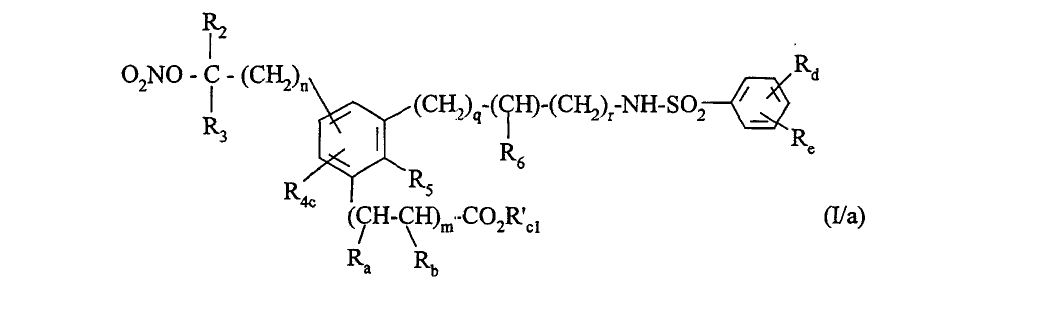 dans laquelle :
R₂, R₃, R₅, R₆, Rₐ, R_{b}, R_{d}, Rₑ, m, n, q et r ont la même signification que dans la formule (I), et
R_{4c} représente un atome d'hydrogène, un groupement alkyle (C₁-C₆) linéaire ou ramifié, phényle éventuellement substitué ou un groupement 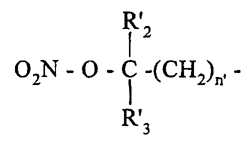 (dans lequel R'₂, R'₃ et n' ont la même signification que précédemment et,
R'c₁ représente un groupement alkyle (C₁-C₆) linéaire ou ramifié (éventuellement substitué par un groupement -O-NO₂)),
**soit** que l'on fait réagir avec du nitrite de terbutyle, pour conduire au composé de formule (I/b), cas particulier des composés de formule (I) : 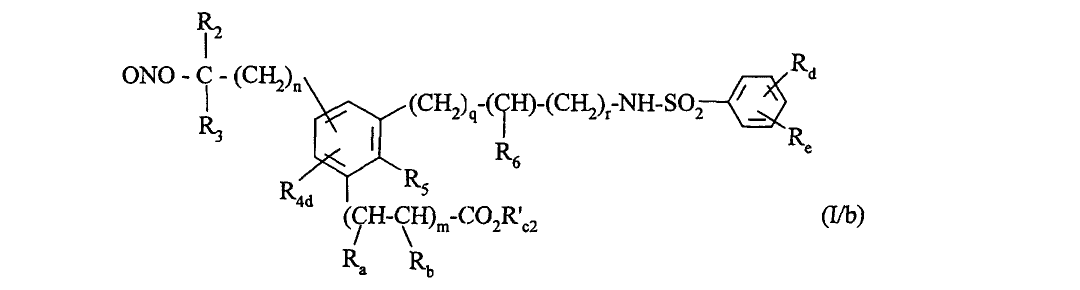 dans laquelle :
R₂, R₃, R₅, R₆, Rₐ, R_{b}, R_{d}, m, n, q et r ont la même signification que précédemment, et
R_{4d} représente un atome d'hydrogène, un groupement alkyle (C₁-C₆) linéaire ou ramifié, phényle éventuellement substitué ou un groupement 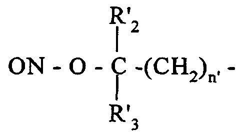 (dans lequel R'₂, R'₃ et n' ont la même signification que précédemment et,
R'c₂ représente un groupement alkyle (C₁-C₆) linéaire ou ramifié (éventuellement substitué par un groupement -O-NO)),
composé de formules (I/a) ou (I/b), dont on transforme, si on le désire, la fonction ester en fonction acide ou amide correspondante,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou une base pharmaceutiquement acceptable.

14. Procédé de préparation des composés de formule (I) selon la revendication 1 tels que p ou p' est égal à 1 et X et X' représentent -NH-CO-, **caractérisé en ce que** l'on utilise comme produit de départ un composé de formule (IV): 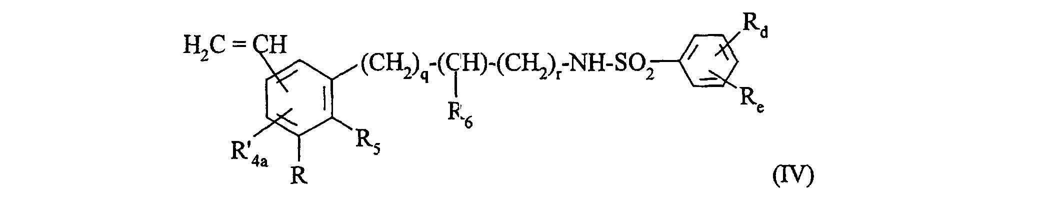 dans laquelle :
R, R_{d}, Rₑ, R₅, R₆, q, r ont la même signification que dans la formule (I),
R'₄ₐ représente un atome d'hydrogène ou un groupement alkyle (C₁-C₆) linéaire ou ramifié (éventuellement substitué par un groupement hydroxy), phényle éventuellement substitué ou un groupement vinyle,
dont on transforme le ou les groupements vinyliques en groupements formyles par coupure oxydative puis en groupements carboxy correspondants,
que l'on fait réagir avec une amine de formule (V): 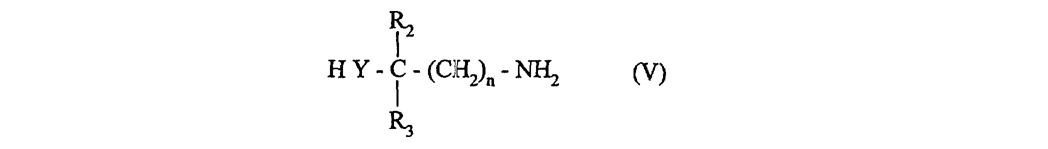 dans laquelle :
R₂, R₃ et n ont la même signification que dans la formule (I)
et Y représente un atome de soufre ou d'oxygène,
pour conduire au composé de formule (VI) : 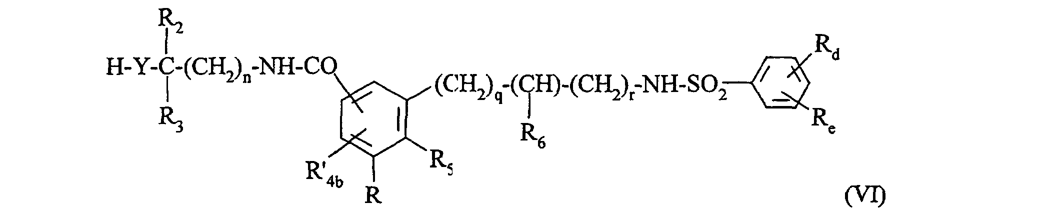 dans laquelle :
R, R_{d}, Rₑ, R₅, R₂, R₃, R₆, Y, n, q et r ont la même signification que précédemment,
R'_{4b} représente un atome d'hydrogène ou un groupement alkyle (C₁-C₆) linéaire ou ramifié (éventuellement substitué par un groupement hydroxy), phényle éventuellement substitué ou un groupement 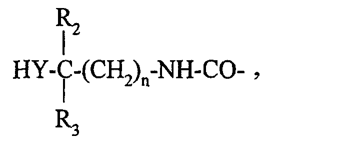 composé de formule (VI) qui est éventuellement transformé lorsque R représente un groupement -CO₂Alk (Alk étant un groupement alkyle (C₁-C₆) linéaire ou ramifié) en aldéhyde correspondant puis qui subit une réaction de Wittig, pour conduire à un composé de formule (VI) dans laquelle le groupement R est remplacé par le groupement -CH=CH-CO₂R'_{c}, R'_{c} représentant un groupement alkyle (C₁-C₆) linéaire ou ramifié éventuellement substitué par un groupement hydroxy,
composé de formule (VI) :
**soit** dont on transforme le ou les groupements hydroxy en un groupement OTs (dans lequel Ts représente le groupement Tosyle) ou en un atome de brome et qui subit l'action du nitrate de tétrabutylammonium ou du nitrate d'argent,
pour conduire au composé de formule (I/c), cas particulier des composés de formule (I) : 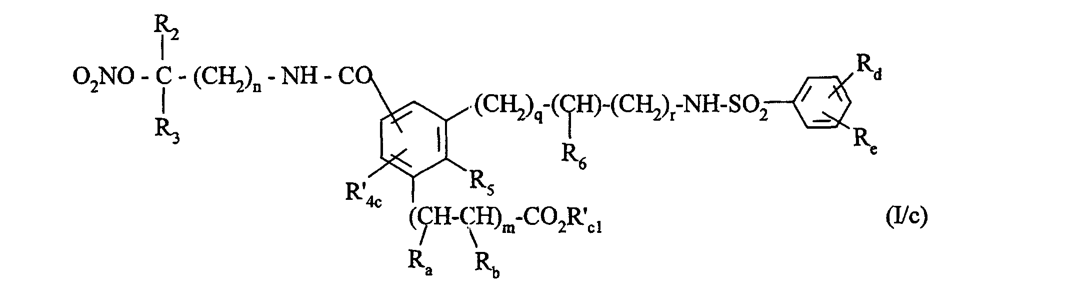 dans laquelle :
R₂, R₃, R₅, R₆, Rₐ, R_{b}, R_{d}, Rₑ, m, n, q et r ont la même signification que dans la formule (I), et
R'_{4c} représente un atome d'hydrogène, un groupement alkyle (C₁-C₆) linéaire ou ramifié (éventuellement substitué par un groupement -ONO₂), phényle éventuellement substitué ou un groupement 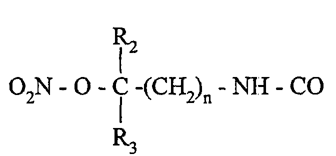 (dans lequel R₂, R₃ et n ont la même signification que précédemment et
R'c₁ représente un groupement alkyle (C₁-C₆) linéaire ou ramifié (éventuellement substitué par un groupement -O-NO₂)),
**soit** que l'on fait réagir avec du nitrite de terbutyle, pour conduire au composé de formule (I/d), cas particulier des composés de formule (I): 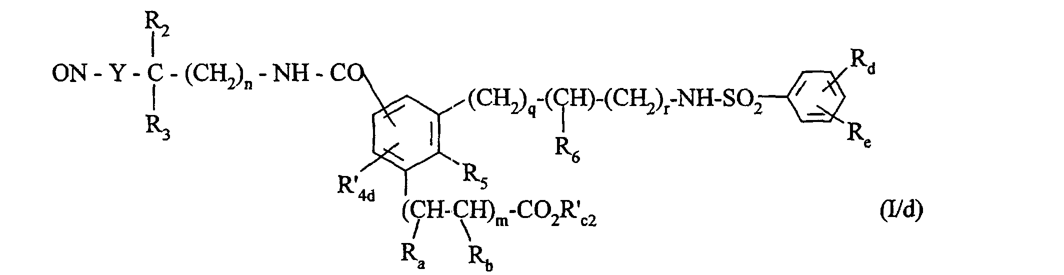 dans laquelle :
Y, R₂, R₃, R₅, R₆, Rₐ, R_{b}, R_{d}, Rₑ, m, n, q et r ont la même signification que précédemment, et
R'_{4d} représente un atome d'hydrogène, un groupement alkyle (C₁-C₆) linéaire ou ramifié (éventuellement substitué par un groupement -O-NO), phényle éventuellement substitué ou un groupement 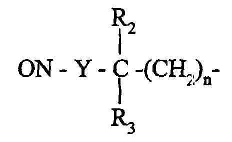 (dans lequel Y, R₂, R₃ et n ont la même signification que précédemment et
R'c₂ représente un groupement alkyle (C₁-C₆) linéaire ou ramifié (éventuellement substitué par un groupement -O-NO)),
composé de formule (I/c) ou (I/d), dont on transforme, si on le désire, la fonction ester en fonction acide ou amide correspondante,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou une base pharmaceutiquement acceptable.

15. Procédé de préparation des composés de formule (I) tels que X représente un atome d'oxygène, **caractérisé en ce que** l'on utilise comme produit de départ un composé de formule (VII): 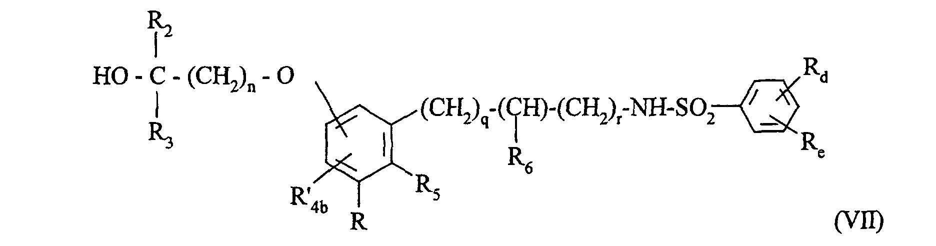 dans laquelle:
R₂, R₃, R₅, R₆, R_{d}, Rₑ, r, q et n sont tels définis dans la formule (I), R représente un groupement 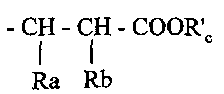 ou COOR'_{c} (dans lesquels R'c représente un groupement alkyle (C₁-C₆)linéaire ou ramifié éventuellement substitué par un groupement hydroxy et Rₐ et R_{b} sont tels que définis dans la formule (1)),
et R'_{4b} représente un atome d'hydrogène, un groupement alkyle (C₁-C₆) linéaire ou ramifié, phényle éventuellement substitué ou un groupement 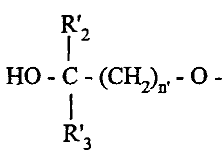 dans lequel n', R'₂ et R'₃ sont tels que définis dans la formule (I)),
composé de formule (VII):
**soit** dont on transforme le ou les groupements hydroxy en un groupement OTs (dans lequel Ts représente un groupement Tosyle) ou en un atome de brome, et qui subit l'action du nitrate de tétrabutylammonium ou du nitrate d'argent,
pour conduire à un composé de formule (I/e), cas particulier des composés de formule (I) 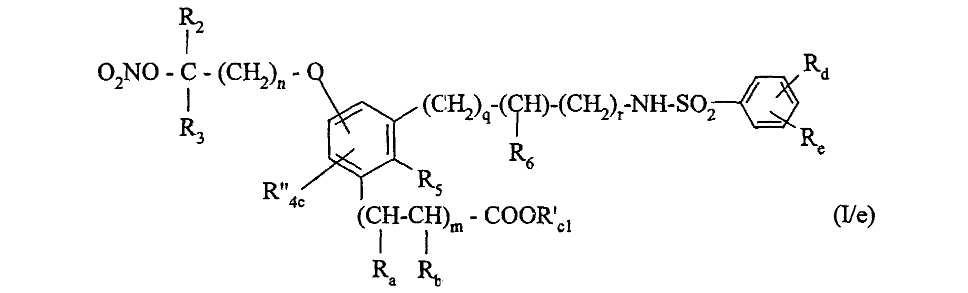 dans laquelle :
R₂, R₃, R₅, R₆, Rₐ, R_{b}, R_{d}, Rₑ, m, n, q et r ont la même signification que dans la formule (I), et
R"_{4c} représente un atome d'hydrogène, un groupement alkyle (C₁-C₆) linéaire ou ramifié, phényle éventuellement substitué ou un groupement 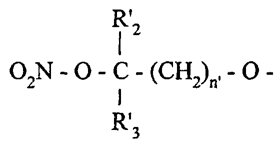 dans lequel R'2, R'3 et n' ont la même signification que précédemment) et R'_{C1} représente un groupement alkyle (C₁-C₆) linéaire on ramifié éventuellement substitué par un groupement -ONO₂,
**soit** que l'on fait réagir avec du nitrite de tertbutyle pour conduire au composé de formule (I/f), cas particulier des composés de formule (I): 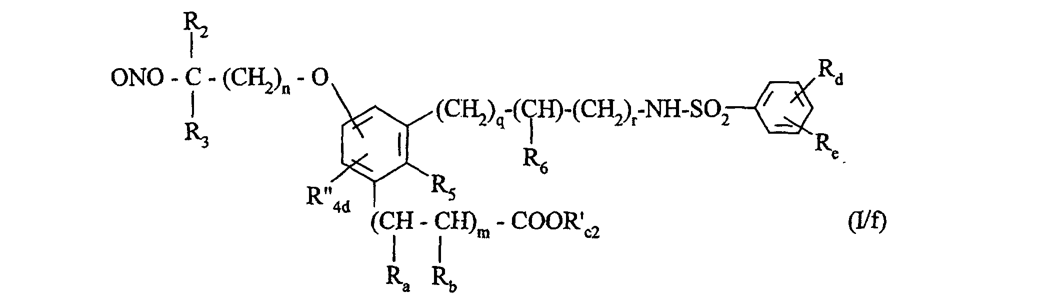 dans laquelle :
R₂, R₃, R₅, R₆, Rₐ, R_{b}, R_{d}, Rₑ, n, m, q et r ont la même signification que dans la formule (I),
R"_{4d} représente un atome d'hydrogène, un groupement alkyle (C₁-C₆) linéaire ou ramifié, phényle éventuellement substitué ou un groupement 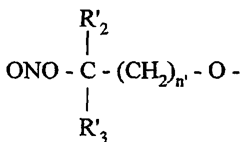 dans lequel R'₂, R'₃ et n' ont la même signification que précédemment et R'_{C2} représente un groupement alkyle (C₁-C₆) linéaire ou ramifié éventuellement substitué par un groupement -O-NO,
composé de formules (I/e) ou (I/f), dont on transforme, si on le désire, la fonction ester en fonction acide ou amide correspondante,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à un acide ou une base pharmaceutiquement acceptable.

16. Compositions pharmaceutiques contenant comme principe actif au moins un composé selon l'une quelconque des revendications 1 à 12, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 12 utiles comme antagonistes des récepteurs TXA₂ et comme donneurs de NO.

## Patentansprüche

1. Verbindung der Formel (I) 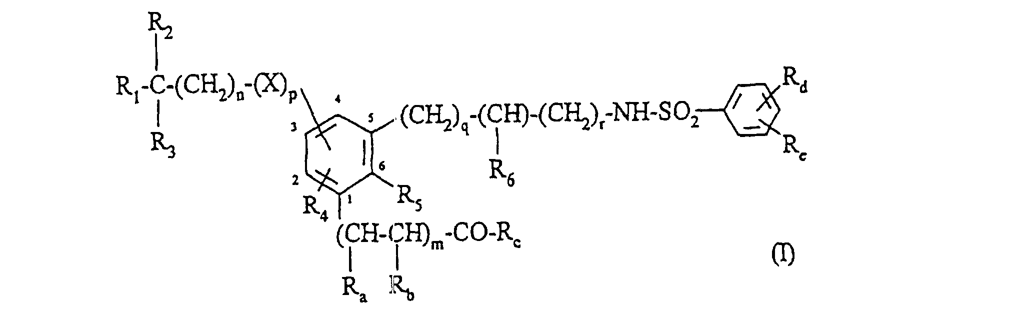 in der:
Rₐ und R_{b}, die gleichartig oder verschieden sind, Wasserstoffatome oder geradkettige oder verzweigte (C₁-C₆)-Alkylgruppen bedeuten oder Rₐ und R_{b} gemeinsam eine Bindung bilden,
R_{c} eine Hydroxygruppe, eine geradkettige oder verzweigte (gegebenenfalls durch eine Gruppe -O-NO₂, -O-NO oder -S-NO substituierte) (C₁-C₆)-Alkoxygruppe, oder eine (gegebenenfalls durch eine oder zwei gleichartige oder verschiedene, geradkettige oder verzweigte (C₁-C₆)-Alkylgruppen oder gegebenenfalls substituierte Phenylgruppen substituierte) Aminogruppe bedeutet,
R₁ ein Wasserstoffatom oder eine Gruppe -O-NO₂, -O-NO oder -S-NO darstellt,
R₂ und R₃, die gleichartig oder verschieden sind, Wasserstoffatome oder geradkettige oder verzweigte (C₁-C₆)-Alkylgruppen oder gegebenenfalls substituierte Phenylgruppen bedeuten,
X ein Sauerstoffatom oder eine Gruppe -NH-CO- darstellt,
m O oder 1 bedeutet,
n eine ganze Zahl bedeutet, so daß die Beziehung 0 ≤ n ≤ 6 gilt,
p 0 oder 1 bedeutet,
R₄ ein Wasserstoffatom, eine geradkettige oder verzweigte (gegebenenfalls durch eine Hydroxygruppe substituierte) (C₁-C₆)-Alkylgruppe, eine gegebenenfalls substituierte Phenylgruppe oder eine Gruppe: 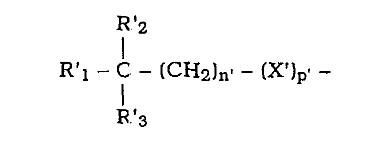 darstellt,
in der:
R'₁ ein Wasserstoffatom oder eine Gruppe: -O-NO₂, -O-NO oder -S-NO,
R'₂ und R'₃, die gleichartig oder verschieden sind, Wasserstoffatome oder geradkettige oder verzweigte (C₁-C₆)-Alkylgruppen oder gegebenenfalls substituierte Phenylgruppen,
X' ein Sauerstoffatom oder eine Gruppe -NH-CO-,
n' eine ganze Zahl, so daß die Beziehung 0 ≤ n ≤ 6 gilt,
p' 0 oder 1 bedeuten,
R₅ ein Wasserstoffatom oder eine geradkettige oder verzweigte (C₁-C₆)-Alkylgruppe darstellt,
R₆ ein Wasserstoffatom oder eine geradkettige oder verzweigte (C₁-C₆)-Alkylgruppe bedeutet
oder R₅ und R₆ gemeinsam eine Kette -(CH₂)ₜ- bilden, in der t 1 oder 2 bedeutet,
q 0, 1 oder 2 bedeutet,
r eine ganze Zahl bedeutet, so daß die Beziehung 0 ≤ r ≤ 6 gilt,
R_{d} und Rₑ, die gleichartig oder verschieden sind, Wasserstoffatome, Halogenatome, geradkettige oder verzweigte (C₁-C₆)-Alkylgruppen, geradkettige oder verzweigte (C₁-C₆)-Alkoxygruppen, Hydroxygruppen oder geradkettige oder verzweigte (C₁-C₆)-Trihalogenalkylgruppen darstellen,
mit der Maßgabe, daß mindestens eine Gruppe -O-NO₂, -O-NO oder -S-NO in R₁, R₄ oder R_{c} vorhanden ist,
und unter einer gegebenenfalls substitulerten Phenylgruppe eine eventuelle Substitution durch ein oder mehrere, gleichartige oder verschiedene Halogenatome oder geradkettige oder verzweigte (C₁-C₆)-Alkylgruppen, geradkettige oder verzweigte (C₁-C₆)-Alkoxygruppen, Hydroxygruppen oder geradkettige oder verzweigte (C₁-C₆)-Trihalogenalkylgruppen zu verstehen ist,
und deren Additionssalze mit einer pharmazeutisch annehmbaren Säure oder Base.

2. Verbindung der Formel (I) nach Anspruch 1, worin R₁ -O-NO₂, -O-NO oder -S-NO bedeutet.

3. Verbindung der Formel (I) nach Anspruch 1, worin Rₐ und R_{b} gleichzeitig Wasserstoffatome bedeuten und m den Wert 1 besitzt.

4. Verbindung der Formel (I) nach Anspruch 1, worin R₅ und R₆ gemeinsam eine Kette -(CH₂)ₜ- bilden.

5. Verbindung der Formel (I) nach Anspruch 1, worin R₄ ein Wasserstoffatom bedeutet.

6. Verbindung der Formel (I) nach Anspruch 1, worin p den Wert 0 besitzt.

7. Verbindung der Formel (I) nach Anspruch 1, worin R_{c} eine Hydroxygruppe oder eine geradkettige oder verzweigte (C₁-C₆)-Alkoxygruppe darstellt.

8. Verbindung der Formel (I) nach Anspruch 1, worin R_{d} ein Halogenatom darstellt, während Rₑ ein Wasserstoffatom bedeutet.

9. Verbindung der Formel (I) nach Anspruch 1, worin R₁ eine Gruppe -O-NO₂ darstellt, während p gleich 0 ist, Rₐ und R_{b} Jeweils Wasserstoffatome bedeuten, während m den Wert 1 besitzt, R₄ ein Wasserstoffatom bedeutet, R₅ und R₆ gemeinsam eine Kette -(CH₂)ₜ-, worin t den Wert 2 besitzt, während q den Wert 1 besitzt, bilden und r den Wert 0 besitzt, R_{d} ein Halogenatom darstellt, während Rₑ ein Wasserstoffatom bedeutet und R_{c} eine Hydroxygruppe oder geradkettige oder verzweigte (C₁-C₆)-Alkoxygruppe darstellt.

10. Verbindung der Formel (I) nach Anspruch 1, nämlich 3-{6-[(4-Chlorphenylsulfonyl)-amino]-2-(2-nitroxyethyl)-5,6,7,8-tetrahydronaphth-1-yl}-propionsäure sowie deren Additionssalze mit einer pharmazeutisch annehmbaren Base.

11. Verbindung der Formel (I) nach Anspruch 1, nämlich 3-{6-[(4-Chlorphenylsulfonyl)-amino]-3-(2-nitroxyethyl)-5,6,7,8-tetrahydronaphth-1-yl}-propionsäure sowie deren Additionssalze mit einer pharmazeutisch annehmbaren Base.

12. Verbindung der Formel (I) nach Anspruch 1, nämlich 3-{6-[(4-Chlorphenylsulfonyl)-amino]-3-nitroxymethyl)-5,6,7,8-tetrahydronaphth-1-yl}-propionsäure sowie deren Additionssalze mit einer pharmazeutisch annehmbaren Base.

13. Verfahren zur Herstellung der Derivate der Formel (I) nach Anspruch 1, worin p = p' = 0 bedeutet, **dadurch gekennzeichnet**, daß man als Ausgangsprodukt eine Verbindung der Formel (II) verwendet: 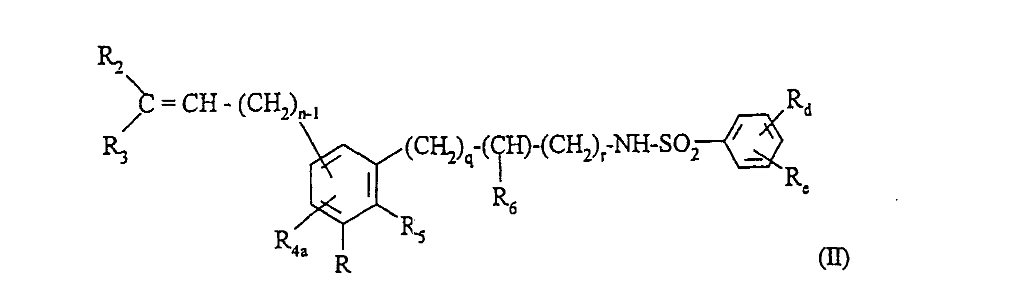 in der:
R_{d}, Rₑ, R₂, R₃, R₅, R₆, n, q und r die bezüglich der Formel (I) angegebenen Bedeutungen besitzen,
R₄ₐ ein Wasserstoffatom, eine geradkettige oder verzweigte (gegebenenfalls durch eine Hydroxygruppe substituierte) (C₁-C₆)-Alkylgruppe, eine gegebenenfalls substituierte Phenylgruppe oder eine Gruppe 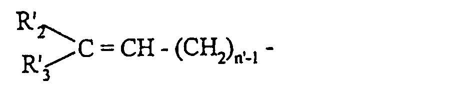 (worin R'₂, R'₃ und n' die bezüglich der Formel (I) angegebenen Bedeutungen besitzen) und
R -CH₂-CH₂-CO₂R'_{c} oder -CO₂R'_{c} (worin R'_{c} eine geradkettige oder verzweigte, gegebenenfalls durch eine Hydroxygruppe substituierte (C₁-C₆)-Alkylgruppe darstellt) bedeuten,
welche man einer geeigneten Oxidationsbehandlung unterwirft, um die Doppelbindung in den entsprechenden Alkohol umzuwandeln, zur Bildung der Verbindung der Formel (III): 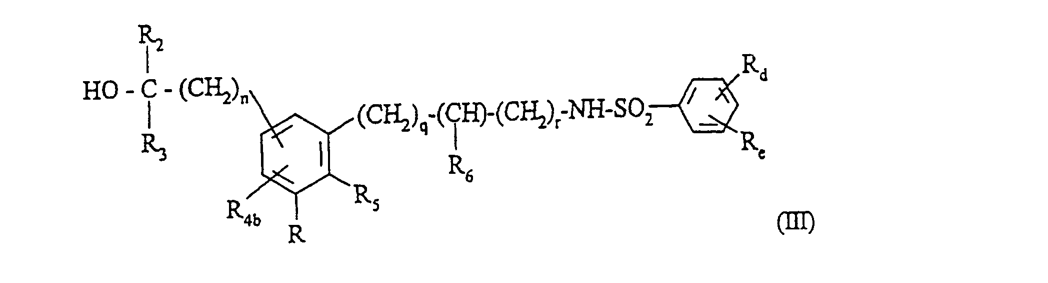 in der:
R, R_{d}, Rₑ, R₂, R₃, R₅, R₆, n, q und r die oben angegebenen Bedeutungen besitzen,
R_{4b} ein Wasserstoffatom, eine geradkettige oder verzweigte (gegebenenfalls durch eine Hydroxygruppe substituierte) (C₁-C₆)-Alkylgruppe, eine gegebenenfalls substituierte Phenylgruppe oder eine Gruppe 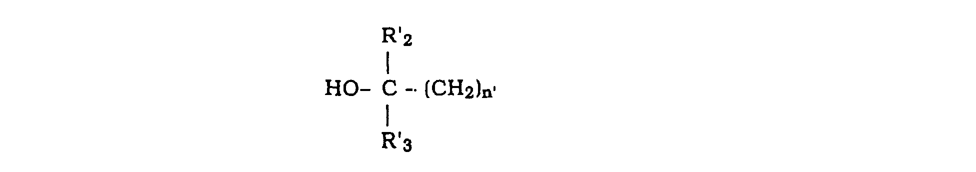 (in der R'₂, R'₃ und n' die oben angegebenen Bedeutungen besitzen) darstellt, welche Verbindung der Formel (III) gegebenenfalls dann, wenn R eine Gruppe -CO₂Alk darstellt (worin Alk für eine geradkettige oder verzweigte (C₁-C₆)-Alkylgruppe steht), in den entsprechenden Aldehyd umgewandelt wird, der einer Wittig-Reaktion unterzogen wird zur Bildung einer Verbindung der Formel (III), in der die Gruppe R durch die Gruppe -CH=CH-CO₂R'_{c} ersetzt ist, worin R'_{c} die oben angegebenen Bedeutungen besitzt,
welche Verbindung der Formel (III) man:
**entweder** einer Behandlung unterwirft zur Umwandlung der Hydroxygruppe(n) in eine Gruppe OTs (worin Ts für die Tosylgruppe steht) oder in ein Bromatom, welches der Einwirkung von Tetrabutylammoniumnitrat oder Silbernitrat unterworfen wird,
zur Bildung der Verbindung der Forme (I/a), einem Sonderfall der Verbindungen der Formel (I): 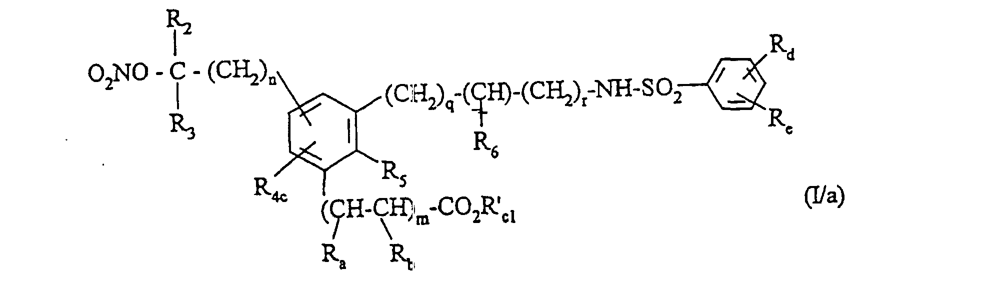 in der:
R₂, R₃, R₅, R₆, Rₐ, R_{b}, R_{d}, Rₑ, m, n, q und r die bezüglich der Formel (I) angegebenen Bedeutungen besitzen und
R_{4c} ein Wasserstoffatom, eine geradkettige oder verzweigte (C₁-C₆)-Alkylgruppe, eine gegebenenfalls substituierte Phenylgruppe oder eine Gruppe 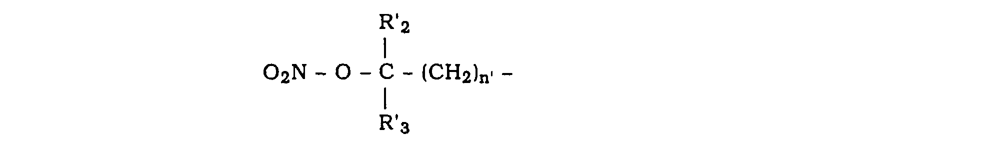 (in der R'₂, R'₃ und n' die oben angegebenen Bedeutungen besitzen) und
R'_{c1} eine geradkettige oder verzweigte (gegebenenfalls durch eine -O-NO₂-Gruppe substituierte) (C₁-C₆)-Alkylgruppe bedeuten,
**oder** mit tert.-Butylnitrit behandelt zur Bildung der Verbindung der Formel (I/b), einem Sonderfall der Verbindungen der Formel (I): 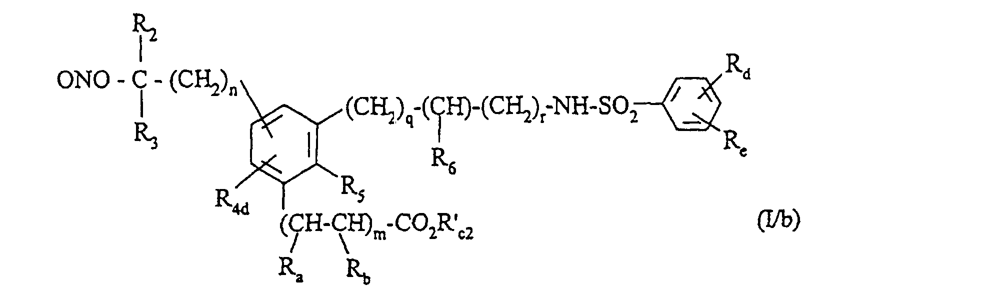 in der:
R₂, R₃, R₅, R₆, Rₐ, R_{b}, R_{d}, m, n, q und r die oben angegebenen Bedeutungen besitzen und
R_{4d} ein Wasserstoffatom, eine geradkettige oder verzweigte (C₁-C₆)-Alkylgruppe, eine gegebenenfalls substituierte Phenylgruppe oder eine Gruppe 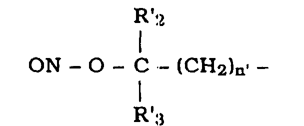 (worin R'₂, R'₃ und n' die oben angegebenen Bedeutungen besitzen) und
R'_{c2} eine geradkettige oder verzweigte (gegebenenfalls durch eine -O-NO-Gruppe substituierte (C₁-C₆)-Alkylgruppe bedeuten,
welche Verbindung der Formeln (I/a) oder (I/b), bei der man gewünschtenfalls die Esterfunktion in die entsprechende Säurefunktion oder Amidfunktion umwandelt,
- man gegebenenfalls mit Hilfe einer klassischen Reinigungsmethode reinigen,
- gegebenenfalls mit Hilfe einer klassischen Trennmethode in die Isomeren auftrennen und
- gewünschtenfalls in ihre Additionssalze mit einer pharmazeutisch annehmbaren Säure oder Base umwandeln kann.

14. Verfahren zur Herstellung der Verbindungen der Formel (I) nach Anspruch 1, worin p oder p' gleich 1 ist und X und X' -NH-CO- bedeuten, **dadurch gekennzeichnet**, daß man als Ausgangsprodukt einer Verbindung der Formel (IV) verwendet: 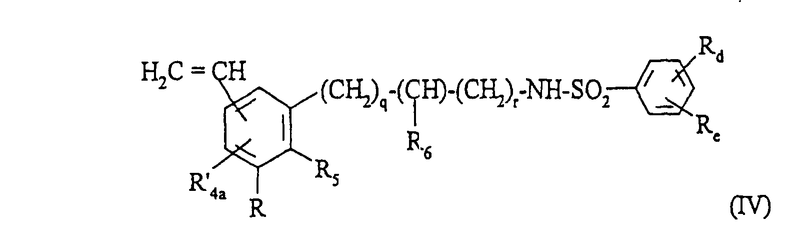 in der:
R, R_{d}, Rₑ, R₅, R₆, q und r die bezüglich der Formel (I) angegebenen Bedeutungen besitzen,
R'₄ₐ ein Wasserstoffatom oder eine geradkettige oder verzweigte (gegebenenfalls durch eine Hydroxygruppe substituierte) (C₁-C₆)-Alkylgruppe, eine gegebenenfalls substituierte Phenylgruppe oder eine Vinylgruppe bedeutet,
deren Vinylgruppe(n) man durch oxidative Spaltung in Formylgruppen und dann in die entsprechenden Carboxygruppen umwandelt,
welche man mit einem Amin der Formel (V) umsetzt: 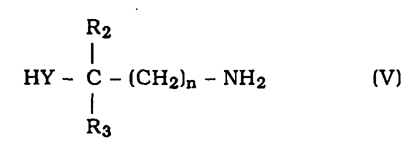 in der R₂, R₃ und n die bezüglich der Formel (I) angegebenen Bedeutungen besitzen und
Y ein Schwefel- oder Sauerstoffatom bedeutet,
zur Bildung der Verbindung der Formel (VI): 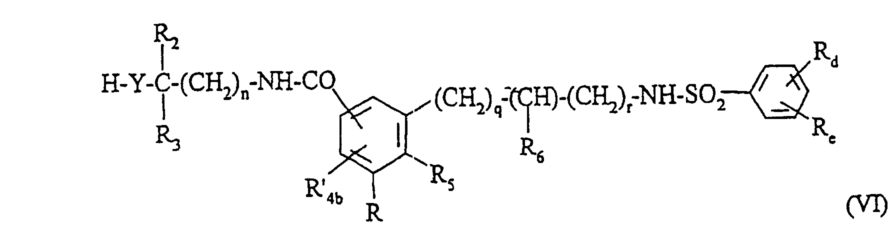 in der:
R, R_{d}, R_{c}, R₅, R₂, R₃, R₆, Y, n, q und r die oben angegebenen Bedeutungen besitzen,
R'_{4b} ein Wasserstoffatom oder eine geradkettige oder verzweigte (gegebenenfalls durch eine Hydroxygruppe substituierte) (C₁-C₆)-Alkylgruppe, eine gegebenenfalls substituierte Phenylgruppe oder eine Gruppe 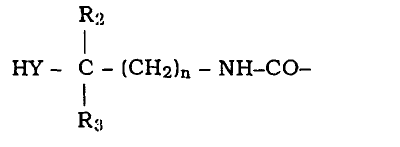 bedeutet,
welche Verbindung der Formel (VI) gegebenenfalls dann, wenn R eine Gruppe -CO₂Alk darstellt (worin Alk für eine geradkettige oder verzweigte (C₁-C₆)-Alkylgruppe steht, in den entsprechenden Aldehyd umgewandelt wird und dann einer Wittig-Reaktion unterworfen wird zur Bildung einer Verbindung der Formel (VI), in der die Gruppe R durch die Gruppe -CH=CH-CO₂R'_{c} ersetzt ist, worin R'_{c} eine geradkettige oder verzweigte, gegebenenfalls durch eine Hydroxygruppe substituierte (C₁-C₆)-Alkylgruppe bedeutet,
welche Verbindung der Formel (VI) man:
**entweder** einer Behandlung unterwirft zur Umwandlung der Hydroxygruppe(n) in eine Gruppe OTs (worin Ts für die Tosylgruppe steht) oder in ein Bromatom, welches der Einwirkung von Tetrabutylammoniumnitrat oder Silbernitrat unterworfen wird,
zur Bildung der Verbindung der Formel (I/c), einem Sonderfall der Verbindungen der Formel (I): 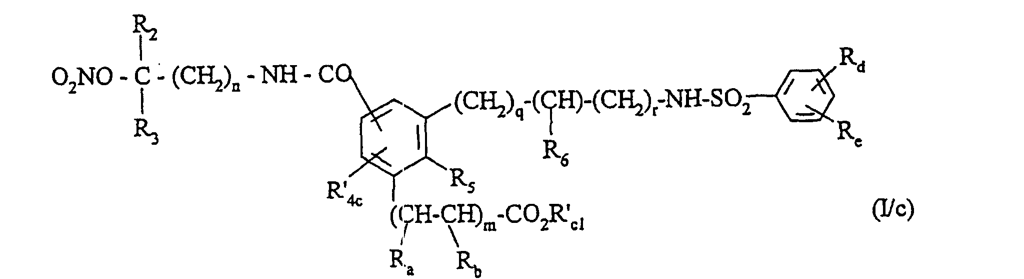 in der:
R₂, R₃, R₅, R₆, Rₐ, R_{b}, R_{d}, Rₑ, m, n, q und r die bezüglich der Formel (I) angegebenen Bedeutungen besitzen und
R'_{4c} ein Wasserstoffatom, eine geradkettige oder verzweigte (gegebenenfalls durch eine Gruppe -ONO₂ substituierte) (C₁-C₆)-Alkylgruppe, eine gegebenenfalls substituierte Phenylgruppe oder eine Gruppe 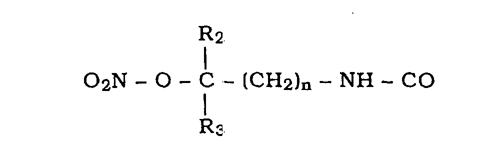 (worin R₂, R₃ und n die oben angegebenen Bedeutungen besitzen) und
R'_{c1} eine geradkettige oder verzweigte (gegebenenfalls durch eine Gruppe -O-NO₂ substituierte) (C₁-C₆)-Alkylgruppe bedeuten,
**oder** mit tert.-Butylnitrit behandelt zur Bildung der Verbindung der Formel (I/d), einem Sonderfall der Verbindungen der Formel (I): 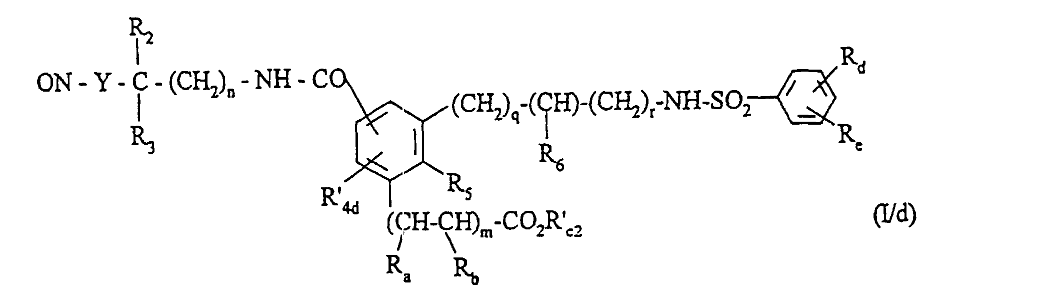 in der:
Y, R₂, R₃, R₅, R₆, Rₐ, R_{b}, R_{d}, Rₑ, m. n, q und r die oben angegebenen Bedeutungen besitzen und
R'_{4d} ein Wasserstoffatom, eine geradkettige oder verzweigte (gegebenenfalls durch eine Gruppe -O-NO substituierte) (C₁-C₆)-Alkylgruppe, eine gegebenenfalls substituierte Phenylgruppe oder eine Gruppe 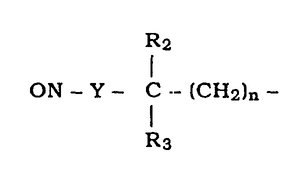 (worin Y, R₂, R₃ und n die oben angegebenen Bedeutungen besitzen) und
R'_{c2} eine geradkettige oder verzweigte (gegebenenfalls durch eine Gruppe -O-NO substituierte) (C₁-C₆)-Alkylgruppe bedeuten,
welche Verbindung der Formel (I/c) oder (I/d), bei der man gewünschtenfalls die Esterfunktion in die entsprechende Säure- oder Amidfunktion umwandelt,
- man gegebenenfalls mit Hilfe einer klassischen Reinigungsmethode reinigt,
- man gegebenenfalls mit Hilfe einer klassischen Trennmethode in die Isomeren auftrennt und
- man gewünschtenfalls in ihre Additionssalze mit einer pharmazeutisch annehmbaren Säure oder Base umwandelt.

15. Verfahren zur Herstellung der Verbindungen der Formel (I), worin X ein Sauerstoffatom darstellt, **dadurch gekennzeichnet**, daß man als Ausgangsprodukt eine Verbindung der Formel (VII) verwendet: 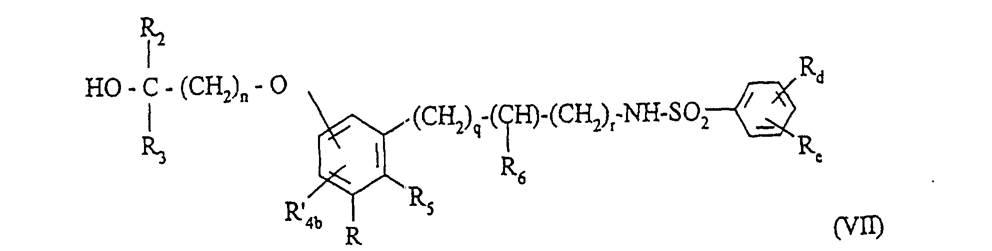 in der:
R₂, R₃, R₅, R₆, R_{d}, Rₑ, r, q und n die bezüglich der Formel (I) angegebenen Bedeutungen besitzen, R eine Gruppe 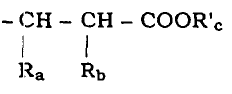 oder COOR'_{c} (worin R'_{c} eine geradkettige oder verzweigte, gegebenenfalls durch eine Hydroxygruppe substituierte (C₁-C₆)-Alkylgruppe darstellt und Rₐ und R_{b} die bezüglich der Formel (I) angegebenen Bedeutungen besitzen)
und R'_{4b} ein Wasserstoffatom, eine geradkettige oder verzweigte (C₁-C₆)-Alkylgruppe, eine gegebenenfalls substituierte Phenylgruppe oder eine Gruppe 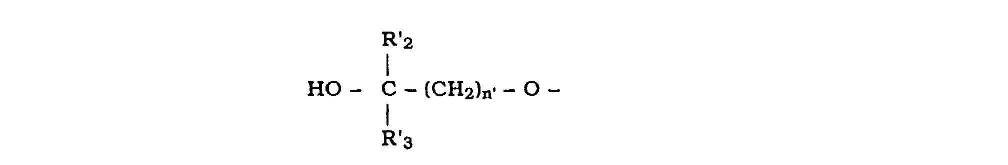 in der n', R'₂ und R'₃ die bezüglich der Formel (I) angegebenen Bedeutungen besitzen, bedeuten,
welche Verbindung der Formel (VII) man:
**entweder** einer Behandlung unterwirft zur Umwandlung der Hydroxygruppe(n) in eine Gruppe OTs (worin Ts eine Tosylgruppe darstellt) oder in ein Bromatom und der Einwirkung von Tetrabutylammoniumnitrat oder Silbernitrat unterwirft, zur Bildung einer Verbindung der Formel (I/e), einem Sonderfall der Verbindungen der Formel (I): 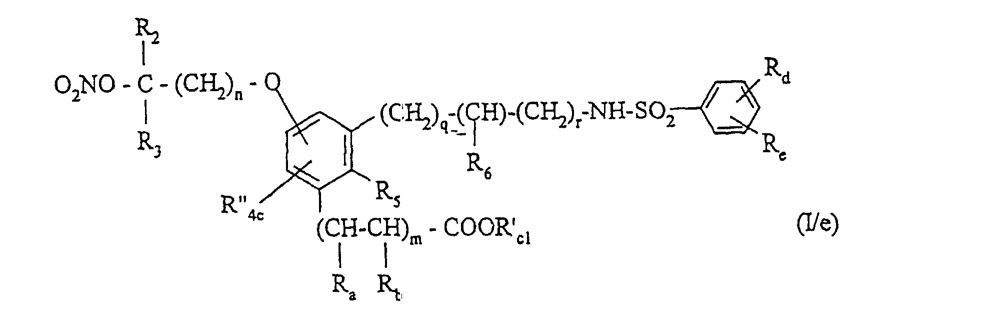 in der:
R₂, R₃, R₅, R₆, Rₐ, R_{b}, R_{d}, Rₑ, m, n, q und r die bezüglich der Formel (I) angegebenen Bedeutungen besitzen und
R"_{4c} ein Wasserstoffatom, eine geradkettige oder verzweigte (C₁-C₆)-Alkylgruppe, eine gegebenenfalls substituierte Phenylgruppe oder eine Gruppe 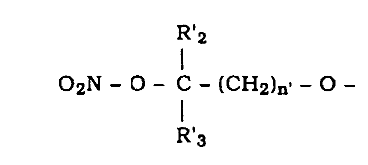 (in der R'₂, R'₃ und n' die oben angegebenen Bedeutungen besitzen) und R'_{c1} eine geradkettige oder verzweigte, gegebenenfalls durch eine Gruppe -ONO₂ substituierte (C₁-C₆)-Alkylgruppe bedeuten,
**oder** man mit tert.-Butylnitrit behandelt zur Bildung der Verbindung der Formel (I/f), einem Sonderfall der Verbindungen der Formel (I): 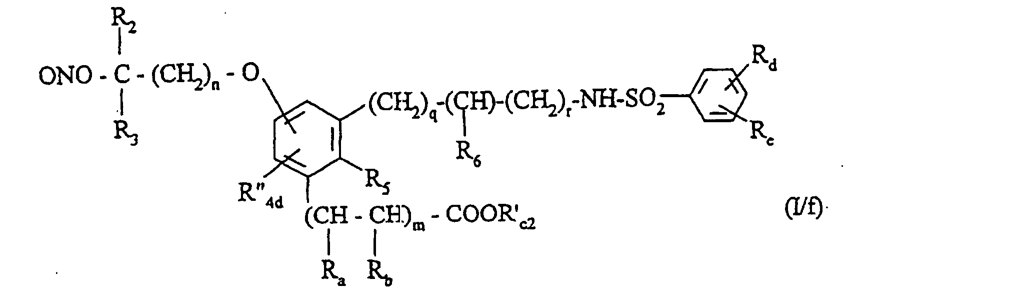 in der:
R₂, R₃, R₅, R₆, Rₐ, R_{b}, R_{d}, Rₑ, n, m, q und r die bezüglich der Formel (I) angegebenen Bedeutungen besitzen,
R"_{4d} ein Wasserstoffatom, eine geradkettige oder verzweigte (C₁-C₆)-Alkylgruppe, eine gegebenenfalls substituierte Phenylgruppe oder eine Gruppe 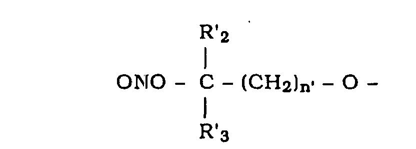 (in der R'₂, R'₃ und n' die oben angegebenen Bedeutungen besitzen) und R'_{c2} eine geradkettige oder verzweigte, gegebenenfalls durch eine Gruppe -O-NO substituierte (C₁-C₆)-Alkylgruppe bedeuten,
welche Verbindung der Formeln (I/e) oder (I/f), deren Esterfunktion man gewünschtenfalls in die entsprechende Säure- oder Amidfunktion umwandelt,
- man gegebenenfalls mit Hilfe einer klassischen Reinigungsmethode reinigen kann,
- man gegebenenfalls mit Hilfe einer klassischen Trennmethode in die Isomeren auftrennen kann,
- man gewünschtenfalls in ihre Additionssalze mit einer pharmazeutisch annehmbaren Säure oder Base umwandelt.

16. Pharmazeutische Zubereitungen enthaltend als Wirkstoff mindestens eine Verbindung nach einem der Ansprüche 1 bis 12 allein oder in Kombination mit einem oder mehreren inerten, nichttoxischen, pharmazeutisch annehmbaren Trägermaterialien oder Bindemitteln.

17. Pharmazeutische Zubereitungen nach Anspruch 16 enthaltend mindestens einen Wirkstoff nach einem der Ansprüche 1 bis 12 als Antagonisten für die Rezeptoren TXA₂ und als NO-Donatoren.

## Claims

1. Compound of formula (I) : 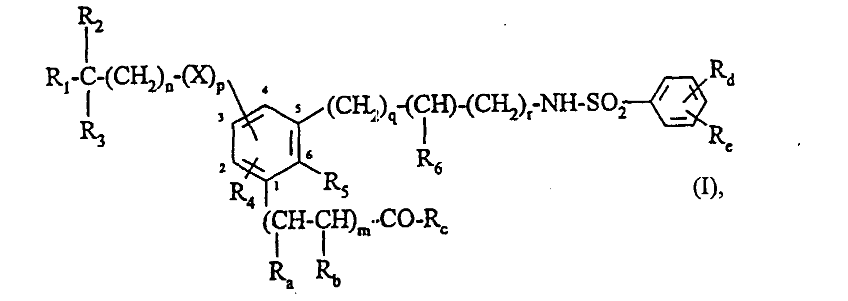 wherein :
Rₐ and R_{b}, which are the same or different, represent a hydrogen atom or a linear or branched (C₁-C₆)alkyl group,
or Rₐ and R_{b} together form a bond,
R_{c} represents a hydroxy group, a linear or branched (C₁-C₆)alkoxy group (optionally substituted by a group -O-NO₂, -O-NO or -S-NO) or an amino group (optionally substituted by one or two identical or different groups: linear or branched (C₁-C₆)alkyl, optionally substituted phenyl),
R₁ represents a hydrogen atom or a group -O-NO₂, -O-NO or -S-NO,
R₂ and R₃, which are the same or different, represent a hydrogen atom or a linear or branched (C₁-C₆)alkyl or optionally substituted phenyl group,
X represents an oxygen atom o:r a group -NH-CO-,
m represents 0 or 1,
n represents a whole number such that 0 ≤ n ≤ 6,
P represents 0 or 1,
R₄ represents a hydrogen atom, a linear or branched (C₁-C₆)alkyl group (optionally substituted by a hydroxy group), an optionally substituted phenyl group or a group: 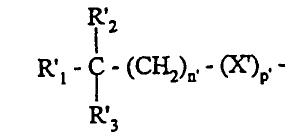 wherein :
R'₁ : represents a hydrogen atom or a group : -O-NO₂, -O-NO or -S-NO,
R'₂ and R'₃, which are the same or different, represent a hydrogen atom or a linear or branched (C₁-C₆)alkyl or optionally substituted phenyl group,
X' represents an oxygen atom or a group -NH-CO-,
n' represents a whole number such that 0 ≤ ≤ 6,
p' represents 0 or 1,
R₅ represents a hydrogen atom or a linear or branched (C₁-C₆)alkyl group,
R₆ represents a hydrogen atom or a linear or branched (C₁-C₆)alkyl group, or R₅ and R₆ together form a -(CH₂)ₜ- chain wherein t represents 1 or 2,
q represents 0, 1 or 2,
r represents a whole number such that 0 ≤ r ≤ 6,
R_{d} and Rₑ, which are the same or different, represent a hydrogen atom, a halogen atom, a linear or branched (C₁-C₆)alkyl group, a linear or branched (C₁-C₆)alkoxy group, a hydroxy group or a linear or branched (C₁-C₆)trihaloalkyl group,
it being understood that at least one group -O-NO₂, -O-NO or -S-NO is present in R₁, R₄ or R_{c},
and that optionally substituted phenyl is understood to mean optionally substituted by one or more, identical or different, halogen atoms or linear or branched (C₁-C₆)alkyl, linear or branched (C₁-C₆)alkoxy, hydroxy or linear or branched (C₁-C₆)trihaloalkyl groups,
and salts thereof with a pharmaceutically acceptable acid or base.

2. Compound of formula (I) according to claim 1, wherein R₁ represents a group -O-NO₂, -O-NO or -S-NO.

3. Compound of formula (I) according to claim 1, wherein Rₐ and R_{b} simultaneously represent a hydrogen atom and m is 1.

4. Compound of formula (I) according to claim 1, wherein R₅ and R₆ together form a chain -(CH₂)ₜ-.

5. Compound of formula (I) according to claim 1, wherein R₄ represents a hydrogen atom.

6. Compound of formula (I) according to claim 1, wherein p is 0.

7. Compound of formula (I) according to claim 1, wherein R_{c} represents a hydroxy group or a linear or branched (C₁-C₆)alkoxy group.

8. Compound of formula (I) according to claim 1, wherein R_{d} represents a halogen atom while Rₑ represents a hydrogen atom.

9. Compound of formula (I) according to claim 1, wherein R₁ represents a group -O-NO₂ while p is 0, each of Rₐ and R_{b} represents a hydrogen atom while m is 1, R₄ represents a hydrogen atom, R₅ and R₆ together form a chain -(CH₂)ₜ- wherein t is 2 while q is 1, and r is 0, R_{d} represents a halogen atom while Rₑ represents a hydrogen atom and R_{c} represents a hydroxy group or a linear or branched (C₁-C₆)alkoxy group.

10. Compound of formula (I) according to claim 1, which is 3-{6-[(4-chlorophenylsulphonyl)amino]-2-(2-nitroxyethyl)-5,6,7,8-tetrahydronaphth-1-yl}propionic acid, and addition salts thereof with a pharmaceutically acceptable base.

11. Compound of formula (I) according to claim 1, which is 3-{6-[(4-chlorophenylsulphonyl)amino]-3-(2-nitroxyethyl)-5,6,7,8-tetrahydronaphth-1-yl}propionic acid, and addition salts thereof with a pharmaceutically acceptable base.

12. Compound of formula (I) according to claim 1, which is 3-{6-[(4-chlorophenylsulphonyl)amino]-3-(2-nitroxymethyl)-5,6,7,8-tetrahydronaphth-1-yl}propionic acid, and addition salts thereof with a pharmaceutically acceptable base.

13. Process for the preparation of compounds of formula (I) according to claim 1 wherein p = p' = 0, **characterised in that** there is used as starting material a compound of formula (II) : 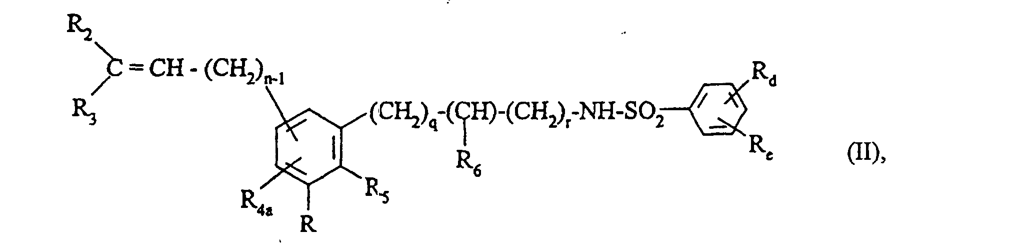 wherein :
R_{d}, Rₑ, R₂, R₃, R₅, R₆, n, q and r are as defined for formula (I),
R₄ₐ represents a hydrogen atom, a linear or branched (C₁-C₆)alkyl group (optionally substituted by a hydroxy group), an optionally substituted phenyl group or a group 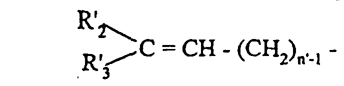 (wherein R'₂, R'₃ and n' are as defined for formula (I)), and R represents -CH₂-CH₂-CO₂R'_{c} or -CO₂R'_{c} (wherein R'_{c} represents a linear or branched (C₁-C₆)alkyl group optionally substituted by a hydroxy group),
which is subjected to appropriate oxidising treatment of the double bond in an appropriate alcohol
to yield the compound of formula (III): 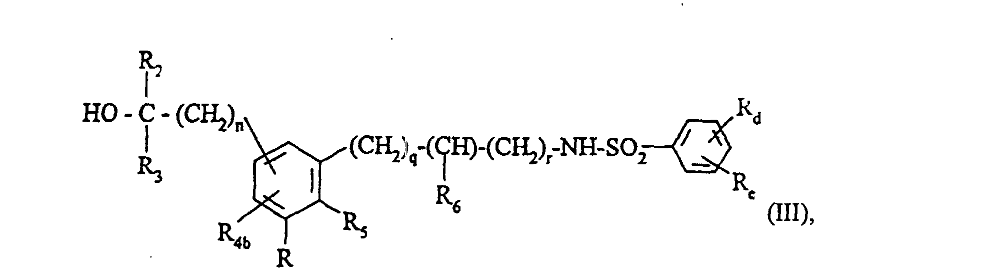 wherein :
R, R_{d}, Rₑ, R₂, R₃, R₅, R₆, n, q and r are as defined hereinbefore,
R_{4b} represents a hydrogen atom, a linear or branched (C₁-C₆)alkyl group (optionally substituted by a hydroxy group), an optionally substituted phenyl group or a group 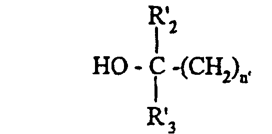 (wherein R'₂, R'₃ and n' are as defined hereinbefore),
which compound of formula (III) is optionally converted, when R represents a group -CO₂Alk (Alk being a linear or branched (C₁-C₆)alkyl group), into the corresponding aldehyde which is then subjected to a Wittig reaction to yield a compound of formula (III) wherein the group R is replaced by the group -CH=CH-CO₂R'_{c}, R'_{c} being as defined hereinbefore,
**either** the hydroxy group(s) of which compound of formula (III) is/are converted into a group OTs (wherein Ts represents the group tosyl) or into a bromine atom and that compound is subjected to the action of tetrabutylammonium nitrate-or silver nitrate
to yield the compound of formula (I/a), a particular case of the compounds of formula (I) : 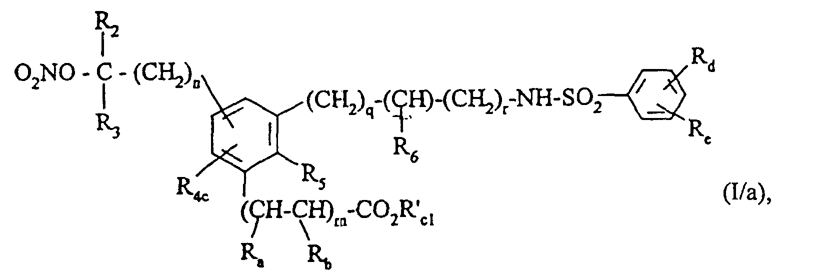 wherein :
R₂, R₃, R₅, R₆, Rₐ, R_{b}, R_{d}, Rₑ, m, n, q and r are as defined for formula (I), and
R_{4c} represents a hydrogen atom, a linear or branched (C₁-C₆)alkyl group, an optionally substituted phenyl group or a group are as defined hereinbefore) and 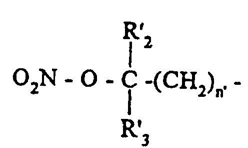 (wherein R'₂, R'₃ and n'
R'_{c1} represents a linear or branched (C₁-C₆)alkyl group (optionally substituted by a group -O-NO₂),
**or** which compound of formula (III) is reacted with tert-butyl nitrite to yield the compound of formula (I/b), a particular case of the compounds of formula (I): 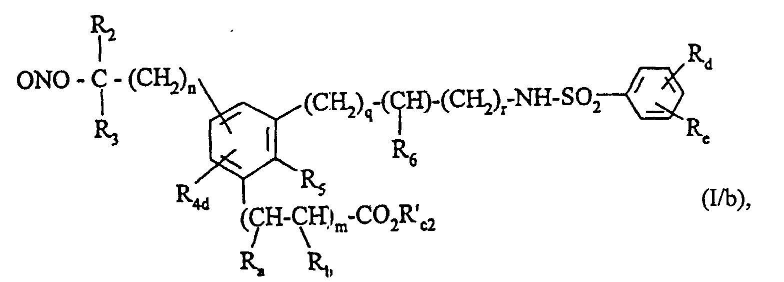 wherein :
R₂, R₃, R₅, R₆, Rₐ, R_{b}, R_{d}, m, n, q and r are as defined hereinbefore, and
R_{4d} represents a hydrogen atom, a linear or branched (C₁-C₆)alkyl group, an optionally substituted phenyl group or a group 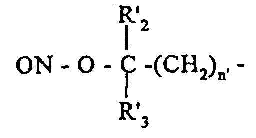 (wherein R'₂, R'₃ and n' are as defined hereinbefore) and
R'_{c2} represents a linear or branched (C₁-C₆)alkyl group (optionally substituted by a group -O-NO),
the ester function of which compound of formula (I/a) or (I/b) is converted, if desired, into a corresponding acid or amide function,
- which compound may be purified, where appropriate, according to a conventional purification technique,
- which compound is separated, where appropriate, into its isomers according to a conventional separation technique,
- which compound is converted, if desired, into its addition salts with a pharmaceutically acceptable acid or base.

14. Process for the preparation of compounds of formula (I) according to claim 1 wherein p or p' is 1 and X and X' represent -NH-CO-, **characterised in that** there is used as starting material a compound of formula (IV) : 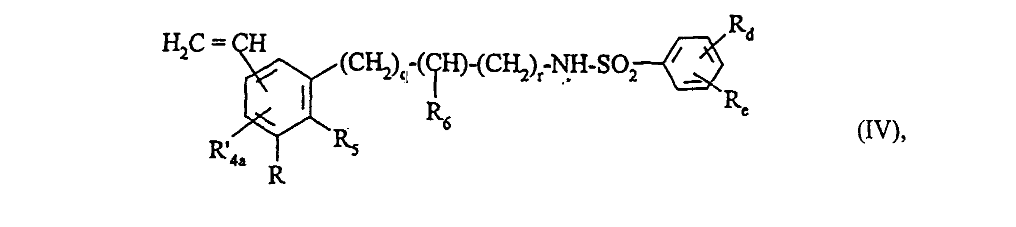 wherein :
R, R_{d}, Rₑ, R₅, R₆, q and r are as defined for formula (I),
R'₄ₐ represents a hydrogen atom or a linear or branched (C₁-C₆)alkyl group (optionally substituted by a hydroxy group), an optionally substituted phenyl group or a vinyl group,
the vinyl group(s) of which is/are converted into formyl groups by oxidative cleavage and then into corresponding carboxy groups,
which is reacted with an amine of formula (V): 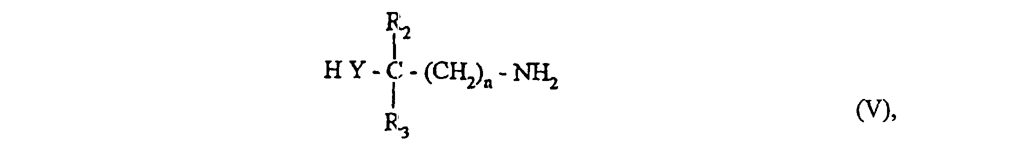 wherein:
R₂, R₃ and n are as defined for formula (I)
and Y represents a sulphur or oxygen atom,
to yield the compound of formula (VI): 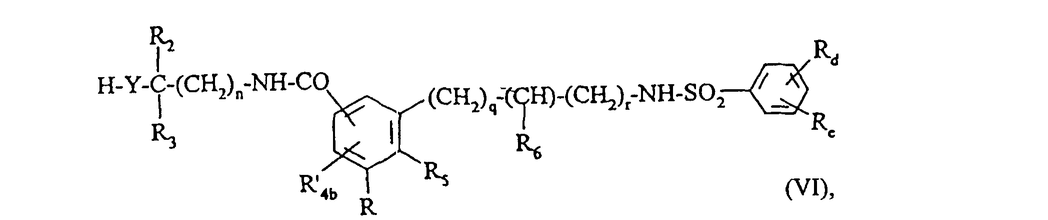 wherein :
R, R_{d}, Rₑ, R₅, R₂, R₃, R₆, Y, n, q and r are as defined hereinbefore,
R'_{4b} represents a hydrogen atom or a linear or branched (C₁-C₆)alkyl group (optionally substituted by a hydroxy group), an optionally substituted phenyl group or a group 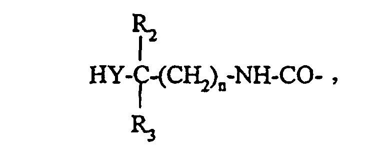 which compound of formula (VI) is optionally converted, when R represents a group -CO₂Alk (Alk being a linear or branched (C₁-C₆)alkyl group), into the corresponding aldehyde which is then subjected to a Wittig reaction to yield a compound of formula (VI) wherein the group R is replaced by the group -CH=CH-CO₂R'_{c}, R'_{c} representing a linear or branched (C₁-C₆)alkyl group optionally substituted by a hydroxy group,
**either** the hydroxy group(s) of which compound of formula (VI) is/are converted into a group OTs (wherein Ts represents the group tosyl) or into a bromine atom and that compound is subjected to the action of tetrabutylammonium nitrate or silver nitrate
to yield the compound of formula (l/c), a particular case of the compounds of formula (I): 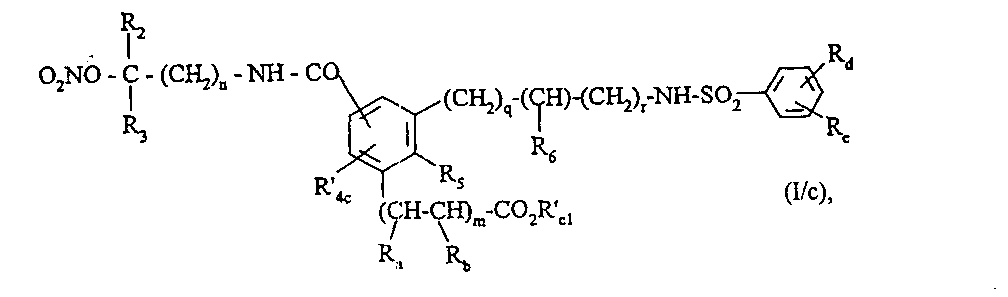 wherein :
R₂, R₃, R₅, R₆, Rₐ, R_{b}, R_{d}, Rₑ, m, n, q and r are as defined for formula (I), and
R'_{4c} represents a hydrogen atom, a linear or branched (C₁-C₆)alkyl group (optionally substituted by a group -ONO₂), an optionally substituted phenyl group or a group 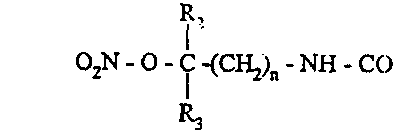 (wherein R₂, R₃ and n are as defined hereinbefore) and
R'_{c1} represents a linear or branched (C₁-C₆)alkyl group (optionally substituted by a group -O-NO₂),
or which compound of formula (VI) is reacted with tert-butyl nitrite to yield the compound of formula (I/d), a particular case of the compounds of formula (I) : 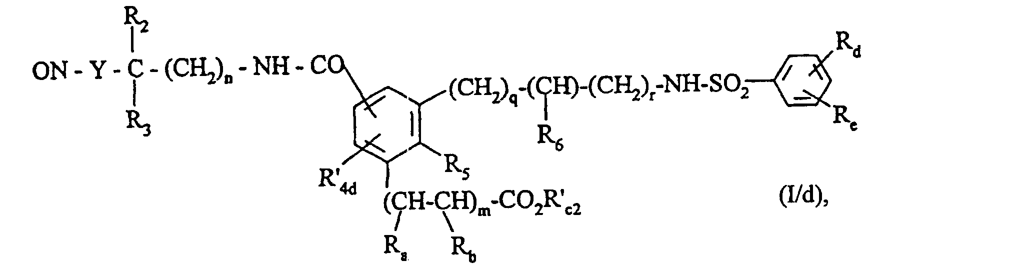 wherein:
Y, R₂, R₃, R₅, R₆, Rₐ, R_{b}, R_{d}, Rₑ, m, n, q and r are as defined hereinbefore, and
R'_{4d} represents a hydrogen atom, a linear or branched (C₁-C₆)alkyl group (optionally substituted by a group -O-NO), an optionally substituted phenyl group or a group 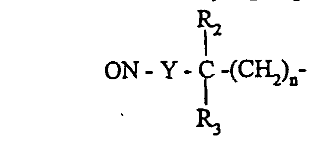 (wherein Y, R₂, R₃ and n are as defined hereinbefore) and
R'_{c2} represents a linear or branched (C₁-C₆)alkyl group (optionally substituted by a group -O-NO),
the ester function of which compound of formula (I/c) or (I/d) is converted, if desired, into a corresponding acid or amide function,
- which compound may be purified, where appropriate, according to a conventional purification technique,
- which compound is separated, where appropriate, into its isomers according to a conventional separation technique,
- which compound is converted, if desired, into its addition salts with a pharmaceutically acceptable acid or base.

15. Process for the preparation of compounds of formula (I) wherein X represents an oxygen atom, **characterised in that** there is used as starting material a compound of formula (VII) : 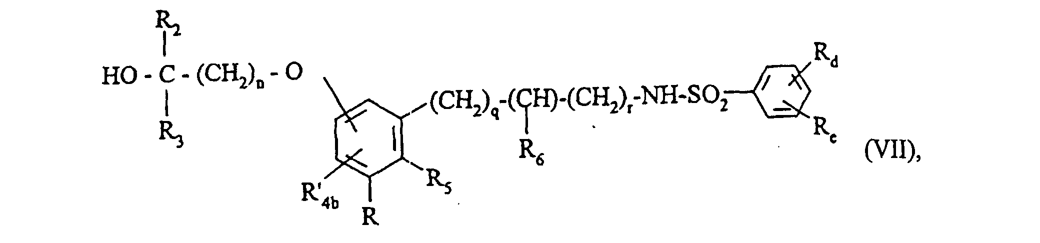 wherein :
R₂, R₃, R₅, R₆, R_{d}, Rₑ, r, q and n are as defined for formula (I), R represents a group 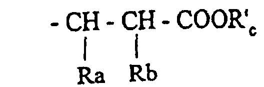 or COOR'_{c} (wherein R'_{c} represents a linear or branched (C₁-C₆)alkyl group optionally substituted by a hydroxy group and Rₐ and R_{b} are as defined for formula (I)),
and R'_{4b} represents a hydrogen atom, a linear or branched (C₁-C₆)alkyl group, an optionally substituted phenyl group or a group 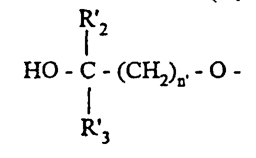 wherein n', R'₂ and R'₃ are as defined for formula (I),
**either** the hydroxy group(s) of which compound of formula (VII) is/are converted into a group OTs (wherein Ts represents the group tosyl) or into a bromine atom and that compound is subjected to the action of tetrabutylammonium nitrate or silver nitrate to yield a compound of formula (I/e), a particular case of the compounds of formula (I): 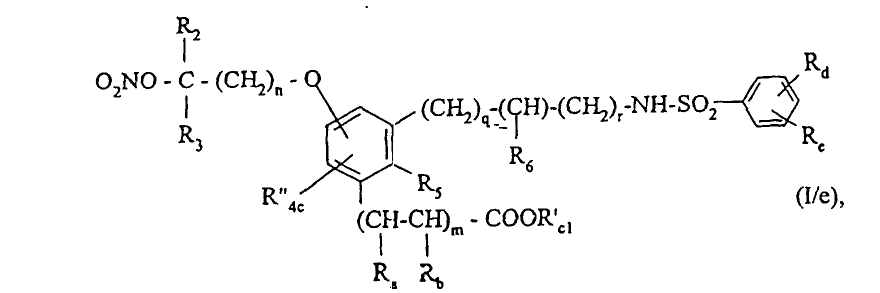 wherein :
R₂, R₃, R₅, R₆, Rₐ, R_{b}, R_{d}, Rₑ, m, n, q and r are as defined for formula (I), and
R"_{4c} represents a hydrogen atom, a linear or branched (C₁-C₆)alkyl group, an optionally substituted phenyl group or a group 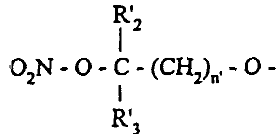 (wherein R'₂, R'₃ and n' are as defined hereinbefore) and R'_{c1} represents a linear or branched (C₁-C₆)alkyl group optionally substituted by a group -ONO₂,
**or** which compound of formula (VII) is reacted with tert-butyl nitrite to yield the compound of formula (I/f), a particular case of the compounds of formula (I): 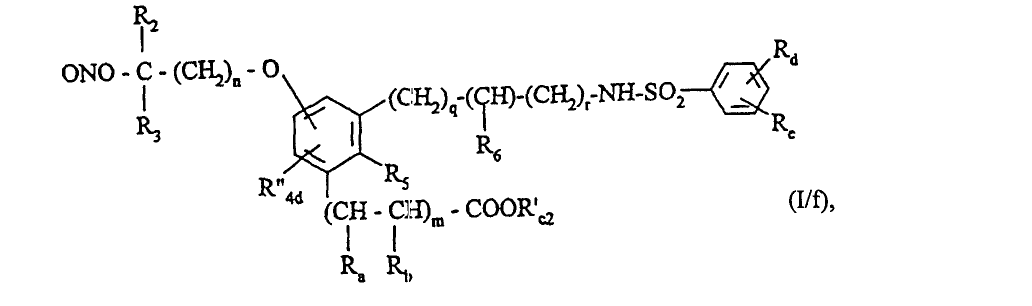 wherein :
R₂, R₃, R₅, R₆, Rₐ, R_{b}, R_{d}, Rₑ, n, m, q and r are as defined for formula (I),
R"_{4d} represents a hydrogen atom, a linear or branched (C₁-C₆)alkyl group, an optionally substituted phenyl group or a group 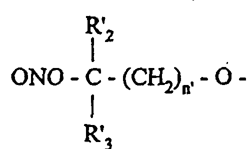 wherein R'₂, R'₃ and n' are as defined hereinbefore, and R'_{c2} represents a linear or branched (C₁-C₆)alkyl group optionally substituted by a group -O-NO,
the ester function of which compound of formula (I/e) or (I/f) is converted, if desired, into a corresponding acid or amide function,
- which compound may be purified, where appropriate, according to a conventional purification technique,
- which compound is separated, where appropriate, into its isomers according to a conventional separation technique,
- which compound is converted, if desired, into its addition salts with a pharmaceutically acceptable acid or base.

16. Pharmaceutical compositions comprising as active ingredient at least one compound according to any one of claims I to 12, alone or in combination with one or more inert, non-toxic, pharmaceutically acceptable excipients or carriers.

17. Pharmaceutical compositions according to claim 16 comprising at least one active ingredient according to any one of claims 1 to 12 for use as antagonists of TXA₂ receptors and as NO donors.
